# EUROPEAN PATENT APPLICATION

(11) **EP 4 324 523 A2**
(43) Date of publication of application: **21.02.2024**
(21) Application number: 23212604.5
(22) Date of filing: 26.03.2018
(51) Int. Cl.: A61P 25/28

(54) **NEW METHODS FOR THE TREATMENT OF MULTIPLE SCLEROSIS**

(30) Priority: 28.03.2017 US 201762477717 P
(62) Divisional of application: 18718215.9
(71) Applicant: Novartis AG, 4056 Basel (CH)
(72) Inventor: SHIMSHEK, Derya, 4002 Basel (CH)
(74) Representative: Mueller, Philippe

(57) **Abstract**

The present invention relates to methods of treating multiple sclerosis, amyotrophic lateral sclerosis (ALS) or Alzheimer's disease in a subject in need of treatment thereof by administering an effective amount of 4-(2-((1R,2R)-2-hydroxycyclohexylamino)benzothiazol-6-yloxy)-N-methylpicolinamide or a pharmaceutically acceptable salt thereof.

## Description

### FIELD OF THE INVENTION

The present invention relates to methods of treating multiple sclerosis, amyotrophic lateral sclerosis (ALS) or Alzheimer's disease (AD) in a subject in need of treatment thereof by administering an effective amount of 4-(2-((1R,2R)-2-hydroxycyclohexylamino)benzothiazol-6-yloxy)-N-methylpicolinamide or a pharmaceutically acceptable salt thereof.

### BACKGROUND OF THE INVENTION

Multiple sclerosis (MS) is a chronic immune-mediated multifocal demyelinating diseases of the central nervous system (CNS) with progressive neurodegeneration. Amyotrophic lateral sclerosis (ALS) is a neurodegenerative disease of motor neurons. Alzheimer's disease (AD) is the most common form of dementia and is caused by accumulation of amyloid-beta leading to amyloid-beta-related neuropathology and loss of cognition. The MS pathology is believed to be mainly driven by the adaptive immune-system and/or autoimmune mechanisms that damage neurons and oligodendrocytes in the CNS leading to white and grey matter degeneration. The relevance of the innate immunity of the CNS in disease initiation and progression has not been extensively investigated. Activated microglia and astrocytes together with neurodegeneration has been observed in MS patients and this neuroinflammatory condition is believed to play an important role in the grey matter damage (for review see (Calabrese et al., 2015; Nylander and Hafler, 2012) (Calabrese et al., 2015 (DOI: 10.1038/nrn3900); Nylander and Hafler, 2012)). Although white matter lesions are the classical hallmarks of MS, the demyelinating events observed in the grey matter might have a more profound contribution to the permanent neurological dysfunction than previously assumed. Microglial activation and demyelination pathologies have also been observed in ALS (Oliveira Santos et al., 2017; Zhou et al., 2017) and AD (Nasrabady et al., 2018; Ropele et al., 2012) patients.

Despite available treatments for MS that primarily contain the peripheral inflammation and help relapsing-remitting MS patients to gain normal neurological function again, a substantial number of MS patients will suffer from irreversible progression of clinical disability. These progressive phases of MS are believed to be the consequence of altered central function of microglia, astrocytes and oligodendroctyes. In this neuroinflammatory milieu, oligodendrocytes and oligodendrocyte precursor cells seem to be unable to exert their proper function, mainly remyelination, and thus further demyelinating events in grey and white matter occur with subsequent axonal pathology. There is a high-unmet medical need in MS patients to stop demyelination and enhance remyelination and prevent irreversible axonal pathology.

### SUMMARY OF THE INVENTION

The present invention relates to new methods of treating multiple sclerosis, amyotrophic lateral sclerosis, Alzheimer's disase which include the administration of an effective amount of a compound of formula (I): 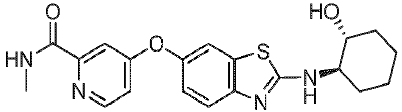 or a pharmaceutically acceptable salt thereof, to a subject in need thereof. In another aspect of the present invention, the pharmaceutically acceptable salt is HCl.

### BRIEF DESCRIPTION OF THE DRAWINGS

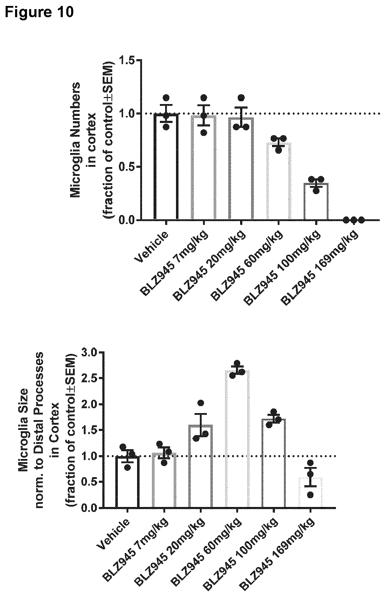

FIG's. 1A-1D provide graphs illustrating magnetic resonance imaging (MRI) contrast in cortex (A), striatum (B) and corpus callosum (C) and Magnetization Transfer Ratio in corpus callosum (D) for the different treatment groups.
FIG. 2 provides example pictures from immunohistological stainings detecting myelin basic protein (MBP) and mature oligodendrocytes positive for GST-π in the cortex for the different treatment groups.
FIG's. 3A-3B provide a quantitative analysis of the immunohistochemistry for MBP (stained area) and GST-π (number of positive cells) in the cortex (A) and striatum (B) normalized to control vehicle.
FIG. 4 provides example pictures from immunohistological stainings detecting myelin oligondendrocyte glycoprotein (MOG) and mature oligodendrocytes positive for GST-π in the corpus callosum (CC) for the different treatment groups.
FIG. 5 provides a quantitative analysis of the immunohistochemistry for MOG (optical density) and GST-π (number of positive cells) and also optical density analysis of Luxol fast blue (LFB) in the corpus callosum normalized to control vehicle.
FIG. 6 provides example pictures from immunohistological stainings detecting the microglia marker Iba1 and glial fibrillary acidic protein (GFAP) astrocytes in the cortex for the different treatment groups.
FIG's. 7A-7C provide quantitative analysis of the immunohistochemistry for Iba1-positive microglia numbers and GFAP-positive astrocyte stained area in the cortex (A), striatum (B) and corpus callosum (C) normalized to control vehicle.
FIG's. 8A-8C provide an evaluation of the clinical disease progression after myelin oligodendrocyte glycoprotein peptide-immunization revealing similar clinical scores (A), disease onset/incidences (B) and weight changes (C).
FIG's. 9A-9C provide magnetic resonance imaging (MRI) contrast in cortex (Figure 9A), striatum (Figure 9B) and corpus callosum (Figure 9C) for the different treatment groups.
FIG's. 10A-10B provides dose-dependent microglia depletion (Iba1-positive microglia numbers) in the cortex of wildtype mice after 5 days of treatment with BLZ945 (p.o., qd) (Figure 10A) and dose-dependent microglia activation (Iba1-positive microglia size normalized to distal microglia processes) in the cortex of wildtype mice after 5 days of treatment with BLZ945 (p.o., qd) (Figure 10B).

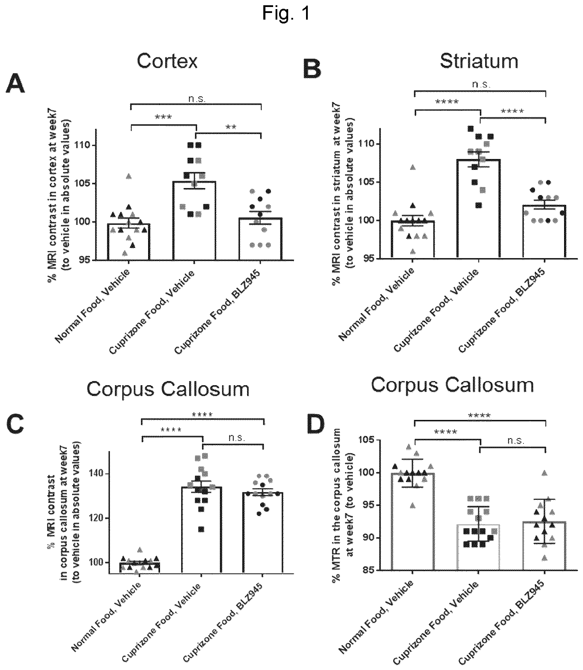

FIG. 1 1 provides pharmacokinetic analysis of BLZ945 in blood and brain.

### DETAILED DESCRIPTION OF THE INVENTION

Multiple sclerosis (MS) is a chronic inflammatory disease affecting the central nervous system (CNS). Amyotrophic lateral sclerosis (ALS) is a neurodegenerative disease of motor neurons and Alzheimer's disease (AD) is characterized by accumulation of amyloid-beta leading to amyloid-beta-related neuropathology and loss of cognition. While multiple effective immunomodulatory therapies for MS exist today, they lack the scope of promoting central nervous system (CNS) repair, in particular remyelination. The innate immune cells of the brain, the microglia, play a pivotal role in regulating myelination processes and the colony-stimulating factor 1 (CSF-1) pathway is a key regulator for microglia differentiation and survival.

The cuprizone model is a general mouse model which is employed to test for compound efficacy in promoting myelination, and thus used to assess potential therapies for treating demyelinating diseases such as multiple sclerosis and amyotrophic lateral sclerosis as well as Alzheimer's disease.

It has surprisingly been found that 4-(2-((1R,2R)-2-hydroxycyclohexylamino)benzothiazol-6-yloxy)-N-methylpicolinamide, a colony stimulating factor 1 (CSF-1) receptor kinase inhibitor, enhanced central remyelination repair mechanisms in a murine cuprizone model and thus could be an effective treatment in subjects with demyelinating diseases such as multiple sclerosis, amyotrophic lateral sclerosis and Alzheimer's disease.

The present invention is directed to a method for the treatment of multiple sclerosis comprising the administration of an effective amount of a compound of formula (I) 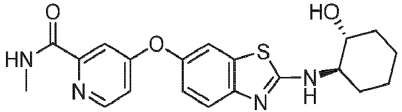 or a pharmaceutically acceptable salt thereof, to a subject in need thereof. In another aspect of the present invention, the pharmaceutically acceptable salt is HCl. In another aspect of the invention a compound of formula (I) is administered as a free base.

The free base and salts of the compound of formula (I) may be prepared for example, according to the procedures given in International Patent Application No. PCT/US2007/066898 filed on April 18, 2007 and published as WO2007/121484 on October 25, 2007. The compound of formula (I) has the chemical name: 4-(2-((1R,2R)-2-hydroxycyclohexylamino)benzothiazol-6-yloxy)-N-methylpicolinamide and is also known as BLZ945.

In a further aspect, the present invention provides for the use of a compound of formula (I) or a pharmaceutically acceptable salt thereof in multiple sclerosis.

In a further aspect the present invention provides a compound of formula (I) or a pharmaceutically acceptable salt thereof for use multiple sclerosis.

In a further aspect, the present invention provides for the use of a compound of formula (I) or a pharmaceutically acceptable salt thereof for the manufacture of a medicament for the treatment of a multiple sclerosis.

In another aspect, the present invention is directed to a method for the treatment of amyotrophic lateral sclerosis comprising the administration of an effective amount of a compound of formula (I) or a pharmaceutically acceptable salt thereof. Preferably, the pharmaceutically acceptable salt is HCl.

In a further aspect, the present invention provides for the use of a compound of formula (I) or a pharmaceutically acceptable salt thereof in amyotrophic lateral sclerosis.

In a further aspect, the present invention provides a compound of formula (I) or a pharmaceutically acceptable salt thereof for use in the treatment of amyotrophic lateral sclerosis.

In a further aspect, the present invention provides for the use of a compound of formula (I) or a pharmaceutically acceptable salt thereof for the manufacture of a medicament for the treatment of Alzheimer's disease.

In another aspect, the present invention is directed to a method for the treatment of Alzheimer's disease comprising the administration of an effective amount of a compound of formula (I) or a pharmaceutically acceptable salt thereof. Preferably, the pharmaceutically acceptable salt is HCl.

In a further aspect, the present invention provides for the use of a compound of formula (I) or a pharmaceutically acceptable salt thereof in Alzheimer's disease.

In a further aspect, the present invention provides a compound of formula (I) or a pharmaceutically acceptable salt thereof for use in the treatment of Alzheimer's disease.

In a further aspect, the present invention provides for the use of a compound of formula (I) or a pharmaceutically acceptable salt thereof for the manufacture of a medicament for the treatment of Alzheimer's disease.

A compound of formula (I) could be useful in the treatment of all phenotypes of multiple sclerosis including, clinically isolated syndrome, relapsing-remitting multiple sclerosis, progressive multiple sclerosis which includes progressive accumulation of disability from onset (primary progressive multiple sclerosis (PPMS)) and progressive accumulation of disability after initial relapsing course (secondary progressive multiple sclerosis (SPMS)).

While it is possible that for use in the treatment methods of the present invention, a compound for formula (I) as well as pharmaceutically acceptable salts thereof, may be administered as the raw chemical, it is possible to present the compound of formula (I) as pharmaceutical composition. Accordingly the invention further provides pharmaceutical compositions which may be administered in the treatment methods of the present invention.

Pharmaceutical compositions of the present invention can be in unit dosage of about 1-1000 mg of active ingredient(s) for a subject of about 50-70 kg, or about 1-500 mg or about 1-250 mg or about 1-150 mg or about 0.5-100 mg, or about 1-50 mg of active ingredients. The therapeutically effective dosage of a compound of formula (I) or a pharmaceutically acceptable salt thereof, the pharmaceutical composition is dependent on the species of the subject, the body weight, age and individual condition, the disorder or disease or the severity thereof being treated. A physician, clinician or veterinarian of ordinary skill can readily determine the effective amount of each of the active ingredients necessary to prevent, treat or inhibit the progress of the disorder or disease.

The above-cited dosage properties are demonstrable *in vitro* and *in vivo* tests using advantageously mammals, e.g., mice, rats, dogs, monkeys or isolated organs, tissues and preparations thereof. The compounds of the present invention can be applied *in vitro* in the form of solutions, *e.g.*, aqueous solutions, and *in vivo* either enterally, parenterally, advantageously intravenously, *e.g.*, as a suspension or in aqueous solution. The dosage *in vitro* may range between about 10⁻³ molar and 10⁻⁹ molar concentrations. A therapeutically effective amount *in vivo* may range depending on the route of administration, between about 0.1-500 mg/kg, or between about 1-100 mg/kg.

Multiple sclerosis (MS) is a chronic inflammatory disease affecting the central nervous system (CNS). While multiple effective immunomodulatory therapies for MS exist today, they lack the scope of promoting central nervous system (CNS) repair, in particular remyelination. In MS the dysfunctional immune system and the resulting chronic (neuro)inflammatory condition induce demyelination of axons. The demyelination is the result of an injury to oligodendrocytes, the myelin-forming cells of the CNS. Based on the findings of the cuprizone-induced demyelination mouse models described in the Biological Assays and Data section given below, administration of 4-(2-((1R,2R)-2-hydroxycyclohexylamino)benzothiazol-6-yloxy)-N-methylpicolinamide or a pharmaceutically acceptable salt thereof could induce remyelination of demylinated axons in patients with multiple sclerosis. The data from the cuprizone mouse models show that the altered microglia morphology observed in naive animals with 60 mg/kg BLZ945 could lead to an enhanced phagocytosis and clearance of myelin debris as well as increasing oligodendrocytes by releasing factors for enhanced oligodendrocyte precursor cell differentiation. Furthermore, the mildly reduced microglia numbers with 60 mg/kg BLZ945 dose in combination with the altered microglia phenotype could protect neurons/axons from being damaged in an inflammatory milieu like in multiple sclerosis. This dose of 60 mg/kg corresponds to about a 300 mg dose in humans.

For the treatment of multiple sclerosis, 4-(2-((1R,2R)-2-hydroxycyclohexylamino)benzothiazol-6-yloxy)-N-methylpicolinamide or a pharmaceutically acceptable salt thereof is administered to a human patient in need thereof at a dose of about 250 mg to about 350 mg per day. In another embodiment the dose is about 275 mg to about 325 mg per day. In another embodiment the dose is about 300 mg. In another embodiment the dose of 4-(2-((1R,2R)-2-hydroxycyclohexylamino)benzothiazol-6-yloxy)-N-methylpicolinamide or a pharmaceutically acceptable salt thereof is administered for 4 to 6 consecutive days and then administration of 4-(2-((1R,2R)-2-hydroxycyclohexylamino)benzothiazol-6-yloxy)-N-methylpicolinamide or a pharmaceutically acceptable salt ceases. In another embodiment, 4-(2-((1R,2R)-2-hydroxycyclohexylamino)benzothiazol-6-yloxy)-N-methylpicolinamide or a pharmaceutically acceptable salt is administered for 4 consecutive days and then administration of 4-(2-((1R,2R)-2-hydroxycyclohexylamino)benzothiazol-6-yloxy)-N-methylpicolinamide or a pharmaceutically acceptable salt ceases. In another embodiment treatment begins at first diagnosis of multiple sclerosis or at the diagnosis of relapsed disease.

The pharmaceutical compositions include a compound of formula (I) or a salt thereof and one or more pharmaceutically acceptable carriers, diluents or excipients. In another aspect, the present invention provides a pharmaceutical composition comprising a compound of formula (I), or a pharmaceutically acceptable salt thereof, and a pharmaceutically acceptable carrier. In a further embodiment, the composition comprises at least two pharmaceutically acceptable carriers, such as those described herein. The pharmaceutical composition can be formulated for particular routes of administration such as oral administration, parenteral administration (e.g. by injection, infusion, transdermal or topical administration), and rectal administration. Topical administration may also pertain to inhalation or intranasal application. The pharmaceutical compositions of the present invention can be made up in a solid form (including, without limitation, capsules, tablets, pills, granules, powders or suppositories), or in a liquid form (including, without limitation, solutions, suspensions or emulsions). Tablets may be either film coated or enteric coated according to methods known in the art. Typically, the pharmaceutical compositions are tablets or gelatin capsules comprising the active ingredient together with one or more of:
a) diluents, *e.g.*, lactose, dextrose, sucrose, mannitol, sorbitol, cellulose and/or glycine;
b) lubricants, *e.g.*, silica, talcum, stearic acid, its magnesium or calcium salt and/or polyethyleneglycol; for tablets also
c) binders, *e.g.*, magnesium aluminum silicate, starch paste, gelatin, tragacanth, methylcellulose, sodium carboxymethylcellulose and/or polyvinylpyrrolidone; if desired
d) disintegrants, *e.g.*, starches, agar, alginic acid or its sodium salt, or effervescent mixtures; and
e) absorbents, colorants, flavors and sweeteners.

As used herein, the term "a therapeutically effective amount" refers to an amount of a drug or pharmaceutical agent that will elicit the biological or medical response of a subject, for example, reduction or inhibition of an enzyme or a protein activity, or ameliorate symptoms, alleviate conditions, slow or delay disease progression, or prevent a disease, etc. In one non-limiting embodiment, the term "a therapeutically effective amount" refers to the amount of drug or pharmaceutical agent that, when administered to a subject, is effective to (1) at least partially alleviate, inhibit, prevent and/or ameliorate a condition, or a disorder or a disease (i) mediated by CSF-1 receptor or (ii) associated with CSF-1 receptor activity, or (iii) characterized by activity (normal or abnormal) of CSF-1 receptor; or (2) reduce or inhibit the activity of CSF-1 receptor; or (3) reduce or inhibit the expression of CSF-1 receptor. In another non-limiting embodiment, the term "a therapeutically effective amount" refers to the amount of drug or pharmaceutical agent that, when administered to a cell, or a tissue, or a non-cellular biological material, or a medium, is effective to at least partially reducing or inhibiting the activity of CSF-1 receptor; or at least partially reducing or inhibiting the expression of CSF-1 receptor.

As used herein, the term "subject" refers to primates (e.g., humans, male or female), dogs, rabbits, guinea pigs, pigs, rats and mice. In certain embodiments, the subject is a primate. In yet other embodiments, the subject is a human.

As used herein, the term "inhibit", "inhibition" or "inhibiting" refers to the reduction or suppression of a given condition, symptom, or disorder, or disease, or a significant decrease in the baseline activity of a biological activity or process.

As used herein, the term "treat", "treating" or "treatment" of any disease or disorder refers to alleviating or ameliorating the disease or disorder (i.e., slowing or arresting the development of the disease or at least one of the clinical symptoms thereof); or alleviating or ameliorating at least one physical parameter or biomarker associated with the disease or disorder, including those which may not be discernible to the patient.

As used herein, the term "prevent", "preventing" or "prevention" of any disease or disorder refers to the prophylactic treatment of the disease or disorder; or delaying the onset or progression of the disease or disorder
The term "pharmaceutically acceptable salts" refers to salts that retain the biological effectiveness and properties of the compound of formula (I) and, which typically are not biologically or otherwise undesirable. In many cases, the compounds of the present invention are capable of forming acid and/or base salts by virtue of the presence of amino and/or carboxyl groups or groups similar thereto.

Pharmaceutically acceptable acid addition salts can be formed with inorganic acids and organic acids.

Inorganic acids from which salts can be derived include, for example, hydrochloric acid, hydrobromic acid, sulfuric acid, nitric acid, phosphoric acid, and the like.

Organic acids from which salts can be derived include, for example, acetic acid, propionic acid, glycolic acid, oxalic acid, maleic acid, malonic acid, succinic acid, fumaric acid, tartaric acid, citric acid, benzoic acid, mandelic acid, methanesulfonic acid, ethanesulfonic acid, toluenesulfonic acid, sulfosalicylic acid, and the like.

Pharmaceutically acceptable base addition salts can be formed with inorganic and organic bases.

Inorganic bases from which salts can be derived include, for example, ammonium salts and metals from columns I to XII of the periodic table. In certain embodiments, the salts are derived from sodium, potassium, ammonium, calcium, magnesium, iron, silver, zinc, and copper; particularly suitable salts include ammonium, potassium, sodium, calcium and magnesium salts.

Organic bases from which salts can be derived include, for example, primary, secondary, and tertiary amines, substituted amines including naturally occurring substituted amines, cyclic amines, basic ion exchange resins, and the like. Certain organic amines include isopropylamine, benzathine, cholinate, diethanolamine, diethylamine, lysine, meglumine, piperazine and tromethamine.

In another aspect, the present invention provides compounds of formula I in acetate, ascorbate, adipate, aspartate, benzoate, besylate, bromide/hydrobromide, bicarbonate/carbonate, bisulfate/sulfate, camphorsulfonate, caprate, chloride/hydrochloride, chlortheophyllonate, citrate, ethandisulfonate, fumarate, gluceptate, gluconate, glucuronate, glutamate, glutarate, glycolate, hippurate, hydroiodide/iodide, isethionate, lactate, lactobionate, laurylsulfate, malate, maleate, malonate, mandelate, mesylate, methylsulphate, mucate, naphthoate, napsylate, nicotinate, nitrate, octadecanoate, oleate, oxalate, palmitate, pamoate, phosphate/hydrogen phosphate/dihydrogen phosphate, polygalacturonate, propionate, sebacate, stearate, succinate, sulfosalicylate, sulfate, tartrate, tosylate trifenatate, trifluoroacetate or xinafoate salt form.

4-(2-((1R,2R)-2-Hydroxycyclohexylamino)benzothiazol-6-yloxy)-N-methylpicolinamide or a pharmaceutically acceptable salt thereof may be administered either simultaneously with, or before or after, one or more other therapeutic agent. 4-(2-((1R,2R)-2-Hydroxycyclohexylamino)benzothiazol-6-yloxy)-N-methylpicolinamide or a pharmaceutically acceptable salt thereof may be administered separately or by the same or different route of administration as the other agents. A therapeutic agent is, for example, a chemical compound, peptide, antibody, antibody fragment or nucleic acid, which is therapeutically active or enhances the therapeutic activity when administered to a patient in combination with a compound of the present invention.

In one embodiment, the invention provides a product comprising 4-(2-((1R,2R)-2-hydroxycyclohexylamino)benzothiazol-6-yloxy)-N-methylpicolinamide or a pharmaceutically acceptable salt thereof and at least one other therapeutic agent as a combined preparation for simultaneous, separate or sequential use in the treatment of multiple sclerosis. Products provided as a combined preparation include a composition comprising 4-(2-((1R,2R)-2-hydroxycyclohexylamino)benzothiazol-6-yloxy)-N-methylpicolinamide or a pharmaceutically acceptable salt thereof and the other therapeutic agent(s) together in the same pharmaceutical composition, or 4-(2-((1R,2R)-2-hydroxycyclohexylamino)benzothiazol-6-yloxy)-N-methylpicolinamide or a pharmaceutically acceptable salt thereof and the other therapeutic agent(s) in separate form, *e.g.* in the form of a kit.

In one embodiment, the invention provides a pharmaceutical composition comprising a 4-(2-((1R,2R)-2-hydroxycyclohexylamino)benzothiazol-6-yloxy)-N-methylpicolinamide or a pharmaceutically acceptable salt thereof and another therapeutic agent(s). Optionally, the pharmaceutical composition may comprise a pharmaceutically acceptable carrier, as described above.

In one embodiment, the invention provides a kit comprising two or more separate pharmaceutical compositions, at least one of which contains 4-(2-((1R,2R)-2-hydroxycyclohexylamino)benzothiazol-6-yloxy)-N-methylpicolinamide or a pharmaceutically acceptable salt thereof. In one embodiment, the kit comprises means for separately retaining said compositions, such as a container, divided bottle, or divided foil packet. An example of such a kit is a blister pack, as typically used for the packaging of tablets, capsules and the like.

The kit of the invention may be used for administering different dosage forms, for example, oral and parenteral, for administering the separate compositions at different dosage intervals, or for titrating the separate compositions against one another. To assist compliance, the kit of the invention typically comprises directions for administration.

In the combination therapies of the invention, 4-(2-((1R,2R)-2-hydroxycyclohexylamino)benzothiazol-6-yloxy)-N-methylpicolinamide or a pharmaceutically acceptable salt thereof and the other therapeutic agent may be manufactured and/or formulated by the same or different manufacturers. Moreover, 4-(2-((1R,2R)-2-hydroxycyclohexylamino)benzothiazol-6-yloxy)-N-methylpicolinamide or a pharmaceutically acceptable salt thereof and the other therapeutic may be brought together into a combination therapy: (i) prior to release of the combination product to physicians (e.g. in the case of a kit comprising the compound of the present invention and the other therapeutic agent); (ii) by the physician themselves (or under the guidance of the physician) shortly before administration; (iii) in the patient themselves, e.g. during sequential administration of the compound of the present invention and the other therapeutic agent.

The other therapeutic agent is:
(1) A corticosteroid, such as oral prednisone and intravenous methylprednisolone
(2) A Beta interferon, such as Avonex^{®}, Betaferon^{®}, Extavia^{®}, Plegridy^{®} or Rebif^{®}
(3) Ocrelizumab (Ocrevus^{®}).
(4) Glatiramer acetate (Copaxone^{®})
(5) Dimethyl fumarate (Tecfidera^{®})
(6) Fingolimod (Gilenya^{®}).
(7) Teriflunomide (Aubagio^{®}).
(8) Natalizumab (Tysabri^{®}).
(9) Alemtuzumab (Lemtrada^{®}) or
(10) Mitoxantrone.

Embodiment 1: A method for the treatment of multiple sclerosis comprising the administration of an effective amount of 4-(2-((1R,2R)-2-hydroxycyclohexylamino)benzothiazol-6-yloxy)-N-methylpicolinamide or a pharmaceutically acceptable salt thereof, to a subject in need thereof.

Embodiment 2: The method according to Embodiment 1 wherein the multiple sclerosis is clinically isolated syndrome.

Embodiment 3: The method according to Embodiment 1 wherein the multiple sclerosis is relapsing-remitting multiple sclerosis.

Embodiment 4: The method according to Embodiment 1 wherein the multiple sclerosis is progressive multiple sclerosis.

Embodiment 5: The method according to Embodiment 1 wherein the multiple sclerosis is primary progressive multiple sclerosis.

Embodiment 6: The method according to Embodiment 1 wherein the multiple sclerosis is secondary progressive multiple sclerosis.

Embodiment 7: The method according to any one of Embodiments 1-6 wherein the pharmaceutically acceptable salt is HCl.

Embodiment 8: The method according to any one of Embodiments 1-6 wherein 4-(2-((1R,2R)-2-hydroxycyclohexylamino)benzothiazol-6-yloxy)-N-methylpicolinamide or a pharmaceutically acceptable salt thereof is administered orally.

Embodiment 9: The method according to Embodiment 8 where the pharmaceutically acceptable salt is HCl.

Embodiment 10: The method according to any one of Embodiments 1-9 wherein the subject is a human.

Embodiment 11: A method for the treatment of multiple sclerosis comprising administration of 4-(2-((1R,2R)-2-hydroxycyclohexylamino)benzothiazol-6-yloxy)-N-methylpicolinamide or a pharmaceutically acceptable salt thereof to a human in need of treatment thereof wherein 4-(2-((1R,2R)-2-hydroxycyclohexylamino)benzothiazol-6-yloxy)-N-methylpicolinamide or a pharmaceutically acceptable salt thereof is administered at a dose of about 250 mg to about 350 mg per day.

Embodiment 12: The method according to Embodiment 11 wherein the dose is about 275 mg to about 325 mg per day.

Embodiment 13: The method according Embodiment 12 wherein the dose is about 300 mg per day.

Embodiment 14: The method according to any one of Embodiments 11-13 wherein 4-(2-((1R,2R)-2-hydroxycyclohexylamino)benzothiazol-6-yloxy)-N-methylpicolinamide or a pharmaceutically acceptable salt thereof is administered for 4 to 6 consecutive days and then administration of 4-(2-((1R,2R)-2-hydroxycyclohexylamino)benzothiazol-6-yloxy)-N-methylpicolinamide or a pharmaceutically acceptable salt thereof ceases.

Embodiment 15: The method according to Embodiment 14 wherein 4-(2-((1R,2R)-2-hydroxycyclohexylamino)benzothiazol-6-yloxy)-N-methylpicolinamide or a pharmaceutically acceptable salt thereof is administered for 4 consecutive days and then administration of 4-(2-((1R,2R)-2-hydroxycyclohexylamino)benzothiazol-6-yloxy)-N-methylpicolinamide or a pharmaceutically acceptable salt thereof ceases.

Embodiment 16: The method according to Embodiment 14 or 15 wherein administration of 4-(2-((1R,2R)-2-hydroxycyclohexylamino)benzothiazol-6-yloxy)-N-methylpicolinamide or a pharmaceutically acceptable salt thereof starts when the human is first diagnosed with multiple sclerosis.

Embodiment 17: A method for the treatment of multiple sclerosis, in a subject in need of treatment thereof, comprising the administration of a pharmaceutical composition comprising an effective amount of 4-(2-((1R,2R)-2-hydroxycyclohexylamino)benzothiazol-6-yloxy)-N-methylpicolinamide or a pharmaceutically acceptable salt thereof and a pharmaceutically acceptable excipient.

Embodiment 18: The method according to Embodiment 17 wherein the multiple sclerosis is clinically isolated syndrome.

Embodiment 19: The method according to Embodiment 17 wherein the multiple sclerosis is relapsing-remitting multiple sclerosis.

Embodiment 20: The method according to Embodiment 17 wherein the multiple sclerosis is progressive multiple sclerosis.

Embodiment 21: The method according to Embodiment 17 wherein the multiple sclerosis is primary progressive multiple sclerosis.

Embodiment 22: The method according to Embodiment 17 wherein the multiple sclerosis is secondary progressive multiple sclerosis.

Embodiment 23: The method according to any one of Embodiments 17-22 wherein the pharmaceutically acceptable salt is HCl.

Embodiment 24: A method according to any one of Embodiments 17-22 wherein 4-(2-((1R,2R)-2-hydroxycyclohexylamino)benzothiazol-6-yloxy)-N-methylpicolinamide or a pharmaceutically acceptable salt thereof is administered orally.

Embodiment 25: The method according to Embodiment 24 where the pharmaceutically acceptable salt is HCl.

Embdoiment 26: The method according to any one of Embodiments 17 to 25 wherein the subject is a human.

Embodiment 27: 4-(2-((1R,2R)-2-hydroxycyclohexylamino)benzothiazol-6-yloxy)-N-methylpicolinamide or a pharmaceutically acceptable salt thereof for use in the treatment of multiple sclerosis.

Embodiment 28 4-(2-((1R,2R)-2-hydroxycyclohexylamino)benzothiazol-6-yloxy)-N-methylpicolinamide or a pharmaceutically acceptable salt thereof for the use according to Embodiment 27 wherein the multiple sclerosis is clinically isolated syndrome.

Embodiment 29: 4-(2-((1R,2R)-2-hydroxycyclohexylamino)benzothiazol-6-yloxy)-N-methylpicolinamide or a pharmaceutically acceptable salt thereof for the use according to Embodiment 27 wherein the multiple sclerosis is relapsing-remitting multiple sclerosis.

Embodiment 30: 4-(2-((1R,2R)-2-hydroxycyclohexylamino)benzothiazol-6-yloxy)-N-methylpicolinamide or a pharmaceutically acceptable salt thereof for the use according to Embodiment 27 wherein the multiple sclerosis is progressive multiple sclerosis.

Embodiment 31: 4-(2-((1R,2R)-2-hydroxycyclohexylamino)benzothiazol-6-yloxy)-N-methylpicolinamide or a pharmaceutically acceptable salt thereof for the use according to Embodiment 27 wherein the multiple sclerosis is primary progressive multiple sclerosis.

Embodiment 32: 4-(2-((1R,2R)-2-hydroxycyclohexylamino)benzothiazol-6-yloxy)-N-methylpicolinamide or a pharmaceutically acceptable salt thereof for the use according to Embodiment 27 wherein the multiple sclerosis is secondary progressive multiple sclerosis.

Embodiment 33: 4-(2-((1R,2R)-2-hydroxycyclohexylamino)benzothiazol-6-yloxy)-N-methylpicolinamide or a pharmaceutically acceptable salt thereof for the use according to any one of Embodiments 27-32 wherein the pharmaceutically acceptable salt is HCl.

Embodiment 34: 4-(2-((1R,2R)-2-hydroxycyclohexylamino)benzothiazol-6-yloxy)-N-methylpicolinamide or a pharmaceutically acceptable salt thereof for the use according to any one of Embodiments 27-32 wherein 4-(2-((1R,2R)-2-hydroxycyclohexylamino)benzothiazol-6-yloxy)-N-methylpicolinamide or a pharmaceutically acceptable salt thereof is administered orally.

Embodiment 35: 4-(2-((1R,2R)-2-hydroxycyclohexylamino)benzothiazol-6-yloxy)-N-methylpicolinamide or a pharmaceutically acceptable salt thereof for the use according to embodiment 34 wherein the pharmaceutically acceptable salt is HCl.

Embodiment 36 : 4-(2-((1R,2R)-2-hydroxycyclohexylamino)benzothiazol-6-yloxy)-N-methylpicolinamide or a pharmaceutically acceptable salt thereof for use in the treatment of multiple sclerosis wherein 4-(2-((1R,2R)-2-hydroxycyclohexylamino)benzothiazol-6-yloxy)-N-methylpicolinamide or a pharmaceutically acceptable salt thereof is administered to a human at a dose of about 250 mg to about 350 mg per day.

Embodiment 37: 4-(2-((1R,2R)-2-hydroxycyclohexylamino)benzothiazol-6-yloxy)-N-methylpicolinamide or a pharmaceutically acceptable salt thereof for the use according to Embodiment 36 wherein the dose is about 275 mg to about 325 mg per day.

Embodiment 38: 4-(2-((1R,2R)-2-hydroxycyclohexylamino)benzothiazol-6-yloxy)-N-methylpicolinamide or a pharmaceutically acceptable salt thereof for the use according to Embodiment 37 wherein the dose is about 300 mg per day.

Embodiment 39: 4-(2-((1R,2R)-2-hydroxycyclohexylamino)benzothiazol-6-yloxy)-N-methylpicolinamide or a pharmaceutically acceptable salt thereof for the use according to any one of Embodiments 36-38 wherein 4-(2-((1R,2R)-2-hydroxycyclohexylamino)benzothiazol-6-yloxy)-N-methylpicolinamide or a pharmaceutically acceptable salt thereof is administered for 4 to 6 consecutive days and then administration of 4-(2-((1R,2R)-2-hydroxycyclohexylamino)benzothiazol-6-yloxy)-N-methylpicolinamide or a pharmaceutically acceptable salt thereof ceases.

Embodiment 40: 4-(2-((1R,2R)-2-hydroxycyclohexylamino)benzothiazol-6-yloxy)-N-methylpicolinamide or a pharmaceutically acceptable salt thereof for the use according to Embodiment 39 wherein 4-(2-((1R,2R)-2-hydroxycyclohexylamino)benzothiazol-6-yloxy)-N-methylpicolinamide or a pharmaceutically acceptable salt thereof is administered for 4 consecutive days and then administration of 4-(2-((1R,2R)-2-hydroxycyclohexylamino)benzothiazol-6-yloxy)-N-methylpicolinamide or a pharmaceutically acceptable salt thereof ceases.

Embodiment 41: 4-(2-((1R,2R)-2-hydroxycyclohexylamino)benzothiazol-6-yloxy)-N-methylpicolinamide or a pharmaceutically acceptable salt thereof for the use according to embodiment 39 or 40 wherein administration of 4-(2-((1R,2R)-2-hydroxycyclohexylamino)benzothiazol-6-yloxy)-N-methylpicolinamide or a pharmaceutically acceptable salt thereof starts when the human is first diagnosed with multiple sclerosis.

Embodiment 42: A pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for use in the treatment of multiple sclerosis.

Embodiment 43: The pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the use according to Embodiment 42 wherein the multiple sclerosis is clinically isolated syndrome.

Embodiment 44: The pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the use according to Embodiment 42 wherein the multiple sclerosis is relapsing-remitting multiple sclerosis.

Embodiment 45: The pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the use according to Embodiment 42 wherein the multiple sclerosis is progressive multiple sclerosis.

Embodiment 46: The pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the use according to Embodiment 42 wherein the multiple sclerosis is primary progressive multiple sclerosis.

Embodiment 47: The pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the use according to Embodiment 42 wherein the multiple sclerosis is secondary progressive multiple sclerosis.

Embodiment 48: The pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the use according to any one of Embodiments 42-47 wherein the pharmaceutically acceptable salt is HCl.

Embodiment 49: The pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the use according to any one of Embodiments 42-47 wherein the composition is administered orally.

Embodiment 50: The pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the use according to Embodiments 49 wherein the pharmaceutically acceptable salt is HCl.

Embodiment 51: Use of 4-(2-((1R,2R)-2-hydroxycyclohexylamino)benzothiazol-6-yloxy)-N-methylpicolinamide or a pharmaceutically acceptable salt thereof in the treatment of multiple sclerosis.

Embodiment 52: Use of 4-(2-((1R,2R)-2-hydroxycyclohexylamino)benzothiazol-6-yloxy)-N-methylpicolinamide or a pharmaceutically acceptable salt thereof for the manufacture of a medicament for the treatment of multiple sclerosis.

Embodiment 53: Use of 4-(2-((1R,2R)-2-hydroxycyclohexylamino)benzothiazol-6-yloxy)-N-methylpicolinamide or a pharmaceutically acceptable salt thereof according to Embodiment 51 or 52 wherein the multiple sclerosis is clinically isolated syndrome.

Embodiment 54: Use of 4-(2-((1R,2R)-2-hydroxycyclohexylamino)benzothiazol-6-yloxy)-N-methylpicolinamide or a pharmaceutically acceptable salt thereof according to Embodiment 51 or 52 wherein the multiple sclerosis is relapsing-remitting multiple sclerosis.

Embodiment 55: Use of 4-(2-((1R,2R)-2-hydroxycyclohexylamino)benzothiazol-6-yloxy)-N-methylpicolinamide or a pharmaceutically acceptable salt thereof according to Embodiment 51 or 52 wherein the multiple sclerosis is progressive multiple sclerosis.

Embodiment 56: Use of 4-(2-((1R,2R)-2-hydroxycyclohexylamino)benzothiazol-6-yloxy)-N-methylpicolinamide or a pharmaceutically acceptable salt thereof according to Embodiment 51 or 52 wherein the multiple sclerosis is primary progressive multiple sclerosis.

Embodiment 57: Use of 4-(2-((1R,2R)-2-hydroxycyclohexylamino)benzothiazol-6-yloxy)-N-methylpicolinamide or a pharmaceutically acceptable salt thereof according to Embodiment 51 or 52 wherein the multiple sclerosis is secondary progressive multiple sclerosis.

Embodiment 58: Use of 4-(2-((1R,2R)-2-hydroxycyclohexylamino)benzothiazol-6-yloxy)-N-methylpicolinamide or a pharmaceutically acceptable salt thereof according to any one of Embodiments 51-57 wherein the pharmaceutically acceptable salt is HCl.

Embodiment 59: Use of 4-(2-((1R,2R)-2-hydroxycyclohexylamino)benzothiazol-6-yloxy)-N-methylpicolinamide or a pharmaceutically acceptable salt thereof according to any one of Embodiments 51-57 wherein 4-(2-((1R,2R)-2-hydroxycyclohexylamino)benzothiazol-6-yloxy)-N-methylpicolinamide is administered orally.

Embodiment 60: Use of 4-(2-((1R,2R)-2-hydroxycyclohexylamino)benzothiazol-6-yloxy)-N-methylpicolinamide or a pharmaceutically acceptable salt thereof in the treatment of multiple sclerosis wherein 4-(2-((1R,2R)-2-hydroxycyclohexylamino)benzothiazol-6-yloxy)-N-methylpicolinamide or a pharmaceutically acceptable salt thereof is administered to a human at a dose of about 250 to about 350 mg per day.

Embodiment 61: The use according to Embodiment 60 wherein the dose is about 275 mg to about 325 mg per day.

Embodiment 62: The use according to Embodiment 61 wherein the dose is about 300 mg per day.

Embodiment 63: The use according to any one of Embodiments 60-62 wherein 4-(2-((1R,2R)-2-hydroxycyclohexylamino)benzothiazol-6-yloxy)-N-methylpicolinamide or a pharmaceutically acceptable salt thereof is administered to a human for 4 to 6 consecutive days and then administration of 4-(2-((1R,2R)-2-hydroxycyclohexylamino)benzothiazol-6-yloxy)-N-methylpicolinamide or a pharmaceutically acceptable salt thereof ceases.

Embodiment 64: The use according to Embodiment 65 wherein 4-(2-((1R,2R)-2-hydroxycyclohexylamino)benzothiazol-6-yloxy)-N-methylpicolinamide or a pharmaceutically acceptable salt thereof is administered to a human for 4 consecutive days and then administration of 4-(2-((1R,2R)-2-hydroxycyclohexylamino)benzothiazol-6-yloxy)-N-methylpicolinamide or a pharmaceutically acceptable salt thereof ceases.

Embodiment 65: The use according Embodiment 63 or 64 wherein administration of 4-(2-((1R,2R)-2-hydroxycyclohexylamino)benzothiazol-6-yloxy)-N-methylpicolinamide or a pharmaceutically acceptable salt thereof starts when the human is first diagnosed with multiple sclerosis.

Embodiment 66: Use of 4-(2-((1R,2R)-2-hydroxycyclohexylamino)benzothiazol-6-yloxy)-N-methylpicolinamide or a pharmaceutically acceptable salt thereof according to Embodiment 65 wherein the pharmaceutically acceptable salt is HCl.

Embodiment 67: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient for the treatment of multiple sclerosis.

Embodiment 68: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient according to Embodiment 67 wherein the multiple sclerosis is clinically isolated syndrome.

Embodiment 69: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient according to Embodiment 67 wherein the multiple sclerosis is relapsing-remitting multiple sclerosis.

Embodiment 70: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient according to Embodiment 67 wherein the multiple sclerosis is progressive multiple sclerosis.

Embodiment 71: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient according to Embodiment 67 wherein the multiple sclerosis is primary progressive multiple sclerosis.

Embodiment 72: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient according to Embodiment 67 wherein the multiple sclerosis is secondary progressive multiple sclerosis.

Embodiment 73: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient according to any one of Embodiments 67-72 wherein the pharmaceutically acceptable salt is HCl.

Embodiment 74: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient according to any one of Embodiments 67-72 wherein the pharmaceutical composition is administered orally.

Embodiment 75: Use of a pharmaceutical composition comprising 4-(2-((1R,2R)-2-hydroxycyclohexylamino)benzothiazol-6-yloxy)-N-methylpicolinamide or a pharmaceutically acceptable salt thereof and at least one pharmaceutically acceptable excipient according to Embodiment 74 wherein the pharmaceutically acceptable salt is HCl.

Embodiment 76: A method for the treatment of multiple sclerosis comprising the administration of an effective amount of 4-(2-((1R,2R)-2-hydroxycyclohexylamino)benzothiazol-6-yloxy)-N-methylpicolinamide or a pharmaceutically acceptable salt thereof in combination with another therapeutic agent, to a subject in need thereof.

Embodiment 77: The method according to embodiment 76 wherein 4-(2-((1R,2R)-2-hydroxycyclohexylamino)benzothiazol-6-yloxy)-N-methylpicolinamide or a pharmaceutically acceptable salt thereof is administered separately or by the same or different route of administration as the other agent.

Embodiment 78: The method according to Embodiment 77 wherein the other therapeutic agent is a corticosteroid, a beta interferon, ocrelizumab. glatiramer acetate, dimethyl fumarate, fingolimod, teriflunomide, natalizumab, alemtuzumab or mitoxantrone.

Embodiment 79: The method according any one of Embodiments 76-78 wherein the multiple sclerosis is clinically isolated syndrome.

Embodiment 80: The method according to any one of Embodiments 76-78 wherein the multiple sclerosis is relapsing-remitting multiple sclerosis.

Embodiment 81: The method according to any one of Embodiments 76-78 wherein the multiple sclerosis is progressive multiple sclerosis.

Embodiment 82: The method according to any one of Embodiments 76-78 wherein the multiple sclerosis is primary progressive multiple sclerosis.

Embodiment 83: The method according to any one of Embodiments 76-78 wherein the multiple sclerosis is secondary progressive multiple sclerosis.

Embodiment 84: The method according to any one of Embodiments 76-83 wherein the pharmaceutically acceptable salt is HCl.

Embodiment 85: 4-(2-((1R,2R)-2-hydroxycyclohexylamino)benzothiazol-6-yloxy)-N-methylpicolinamide or a pharmaceutically acceptable salt thereof in combination with another therapeutic agent for use in the treatment of multiple sclerosis.

Embodiment 86: 4-(2-((1R,2R)-2-hydroxycyclohexylamino)benzothiazol-6-yloxy)-N-methylpicolinamide or a pharmaceutically acceptable salt thereof for the use according to Embodiment 85 wherein 4-(2-((1R,2R)-2-hydroxycyclohexylamino)benzothiazol-6-yloxy)-N-methylpicolinamide or a pharmaceutically acceptable salt thereof is administered separately or by the same or different route of administration as the other agent.

Embodiment 87: 4-(2-((1R,2R)-2-hydroxycyclohexylamino)benzothiazol-6-yloxy)-N-methylpicolinamide or a pharmaceutically acceptable salt thereof for the use according to Embodiment 85 or 86 wherein the other therapeutic agent is a corticosteroid, a beta interferon, ocrelizumab. glatiramer acetate, dimethyl fumarate, fingolimod, teriflunomide, natalizumab, alemtuzumab or mitoxantrone.

Embodiment 88: 4-(2-((1R,2R)-2-hydroxycyclohexylamino)benzothiazol-6-yloxy)-N-methylpicolinamide or a pharmaceutically acceptable salt thereof for the use according to any one of Embodiments 85-87 wherein the multiple sclerosis is clinically isolated syndrome.

Embodiment 89: 4-(2-((1R,2R)-2-hydroxycyclohexylamino)benzothiazol-6-yloxy)-N-methylpicolinamide or a pharmaceutically acceptable salt thereof for the use according to any one of Embodiments 85-87 wherein the multiple sclerosis is relapsing-remitting multiple sclerosis.

Embodiment 90: 4-(2-((1R,2R)-2-hydroxycyclohexylamino)benzothiazol-6-yloxy)-N-methylpicolinamide or a pharmaceutically acceptable salt thereof for use according to any one of Embodiments 85-87 wherein the multiple sclerosis is progressive multiple sclerosis.

Embodiment 91: 4-(2-((1R,2R)-2-hydroxycyclohexylamino)benzothiazol-6-yloxy)-N-methylpicolinamide or a pharmaceutically acceptable salt thereof for the use according to any one of Embodiments 85-87 wherein the multiple sclerosis is primary progressive multiple sclerosis.

Embodiment 92: 4-(2-((1R,2R)-2-hydroxycyclohexylamino)benzothiazol-6-yloxy)-N-methylpicolinamide or a pharmaceutically acceptable salt thereof for the use according to any one of Embodiments 85-87 wherein the multiple sclerosis is secondary progressive multiple sclerosis.

Embodiment 93: 4-(2-((1R,2R)-2-hydroxycyclohexylamino)benzothiazol-6-yloxy)-N-methylpicolinamide or a pharmaceutically acceptable salt thereof for the use according to any one of Embodiments 85-92 wherein the pharmaceutically acceptable salt is HCl.

Embodiment 94: Use of 4-(2-((1R,2R)-2-hydroxycyclohexylamino)benzothiazol-6-yloxy)-N-methylpicolinamide or a pharmaceutically acceptable salt thereof in combination with another therapeutic agent in the treatment of multiple sclerosis.

Embodiment 95: The use according to Embodiment 94 wherein 4-(2-((1R,2R)-2-hydroxycyclohexylamino)benzothiazol-6-yloxy)-N-methylpicolinamide or a pharmaceutically acceptable salt thereof is administered separately or by the same or different route of administration as the other agent.

Embodiment 96: The use according to Embodiment 94 or 95 wherein the other therapeutic agent is a corticosteroid, a beta interferon, ocrelizumab. glatiramer acetate, dimethyl fumarate, fingolimod, teriflunomide, natalizumab, alemtuzumab or mitoxantrone.

Embodiment 97: Use of 4-(2-((1R,2R)-2-hydroxycyclohexylamino)benzothiazol-6-yloxy)-N-methylpicolinamide or a pharmaceutically acceptable salt thereof according to any one of Embodiments 94-96 wherein the multiple sclerosis is clinically isolated syndrome.

Embodiment 98: Use of 4-(2-((1R,2R)-2-hydroxycyclohexylamino)benzothiazol-6-yloxy)-N-methylpicolinamide or a pharmaceutically acceptable salt thereof according to any one of Embodiments 94-96 wherein the multiple sclerosis is relapsing-remitting multiple sclerosis.

Embodiment 99: Use of 4-(2-((1R,2R)-2-hydroxycyclohexylamino)benzothiazol-6-yloxy)-N-methylpicolinamide or a pharmaceutically acceptable salt thereof according to any one of Embodiments 94-96 wherein the multiple sclerosis is progressive multiple sclerosis.

Embodiment 100: Use of 4-(2-((1R,2R)-2-hydroxycyclohexylamino)benzothiazol-6-yloxy)-N-methylpicolinamide or a pharmaceutically acceptable salt thereof according to any one of Embodiments 94-96 wherein the multiple sclerosis is primary progressive multiple sclerosis.

Embodiment 101: Use of 4-(2-((1R,2R)-2-hydroxycyclohexylamino)benzothiazol-6-yloxy)-N-methylpicolinamide or a pharmaceutically acceptable salt thereof according to any one of Embodiments 94-96 wherein the multiple sclerosis is secondary progressive multiple sclerosis.

Embodiment 102: Use of 4-(2-((1R,2R)-2-hydroxycyclohexylamino)benzothiazol-6-yloxy)-N-methylpicolinamide or a pharmaceutically acceptable salt thereof according to any one of Embodiments 94-101 wherein the pharmaceutically acceptable salt is HCl.

Embodiment 103: A method for the treatment of amyotrophic lateral sclerosis comprising the administration of an effective amount of 4-(2-((1R,2R)-2-hydroxycyclohexylamino)benzothiazol-6-yloxy)-N-methylpicolinamide or a pharmaceutically acceptable salt thereof, to a subject in need thereof.

Embodiment 104: The method according to embodiment 103 wherein the subject is a primate.

Embodiment 105: The method according to embodiment 104 wherein the subject is a human.

Embodiment 106: The method according to any one of embodiments 103 to 105 wherein 4-(2-((1R,2R)-2-hydroxycyclohexylamino)benzothiazol-6-yloxy)-N-methylpicolinamide is administered as a free base.

Embodiment 107: The method according to any one of Embodiments 103-105 wherein the pharmaceutically acceptable salt is HCl.

Embodiment 108: The use of 4-(2-((1R,2R)-2-hydroxycyclohexylamino)benzothiazol-6-yloxy)-N-methylpicolinamide or a pharmaceutically acceptable salt thereof in amyotrophic lateral sclerosis.

Embodiment 109: 4-(2-((1R,2R)-2-hydroxycyclohexylamino)benzothiazol-6-yloxy)-N-methylpicolinamide or a pharmaceutically acceptable salt thereof for use in the treatment of amyotrophic lateral sclerosis
Embodiment 110: The use of 4-(2-((1R,2R)-2-hydroxycyclohexylamino)benzothiazol-6-yloxy)-N-methylpicolinamide or a pharmaceutically acceptable salt thereof for the manufacture of a medicament for the treatment of amyotrophic lateral sclerosis.

Embodiment 111: A method for the treatment of Alzheimer's disease comprising the administration of an effective amount of 4-(2-((1R,2R)-2-hydroxycyclohexylamino)benzothiazol-6-yloxy)-N-methylpicolinamide or a pharmaceutically acceptable salt thereof, to a subject in need thereof.

Embodiment 112: The method according to embodiment 111 wherein the subject is a primate.

Embodiment 113: The method according to embodiment 112 wherein the subject is a human.

Embodiment 114: The method according to any one of embodiments 111 to 113 wherein 4-(2-((1R,2R)-2-hydroxycyclohexylamino)benzothiazol-6-yloxy)-N-methylpicolinamide is administered as a free base.

Embodiment 115: The method according to any one of Embodiments 111-113 wherein the pharmaceutically acceptable salt is HCl.

Embodiment 116: The use of 4-(2-((1R,2R)-2-hydroxycyclohexylamino)benzothiazol-6-yloxy)-N-methylpicolinamide or a pharmaceutically acceptable salt thereof in Alzheimer's disease.

Embodiment 117: 4-(2-((1R,2R)-2-hydroxycyclohexylamino)benzothiazol-6-yloxy)-N-methylpicolinamide or a pharmaceutically acceptable salt thereof for use in the treatment of Alzheimer's disease.

Embodiment 118: The use of 4-(2-((1R,2R)-2-hydroxycyclohexylamino)benzothiazol-6-yloxy)-N-methylpicolinamide or a pharmaceutically acceptable salt thereof for the manufacture of a medicament for the treatment of Alzheimer's disease.

### Biological Assays and Data

In the Examples described below, 4-(2-((1R,2R)-2-hydroxycyclohexylamino)benzothiazol-6-yloxy)-N-methylpicolinamide HCl salt was synthesized using known methods in the art. In the assays and results that follow 4-(2-((1R,2R)-2-hydroxycyclohexylamino)benzothiazol-6-yloxy)-N-methylpicolinamide HCl salt is referred to or labeled as BLZ945.

### Example 1 Cuprizone-induced demyelination in mice, treatment and necropsy

Studies described below were approved by the Swiss Cantonal Veterinary Authority of Basel City, Switzerland, under the license number 2711. Mice (C57BL/6J) were commercially purchased from Charles River Laboratories, Germany or obtained from Novartis Pharma AG breeding colonies (8-9 weeks old, females). All the animals were allowed to adapt for 7 days prior the start of the experiment and housed in IVC racks (max. 4 mice/XJ Type cage). The animals were given access to food and water ad libitum. Animals were treated with cuprizone for 5 weeks. Cuprizone (Bis(cyclohexanone)oxaldihydrazone, Sigma-Aldrich) was mixed into rodent food pellets (0.2% w/w) by Provimi Kliba AG, Switzerland. Therapeutic treatment of animals after cuprizone was performed for 2 weeks on normal food with 169 mg/kg of BLZ945, qd, p.o. 10ml/kg. BLZ945 was prepared in vehicle consisting of 0.5% methylcellulose in water and 0.1% Tween-80. Before killing 3h after last BLZ945 dosing animals were perfused transcardially by phosphate-buffered saline (PBS), brains isolated and fixed in 4% paraformaldehyde (PFA) for 48h at 4°C.

### Magnetic Resonance Imaging (MRI)

Measurements were performed with a Biospec 70/30 spectrometer (Bruker Medical Systems, Ettlingen, Germany) operating at 7 T. The operational software of the scanner was Paravision 5.1 (Bruker). Images were acquired from anesthetized, spontaneously breathing animals using a mouse brain circularly polarized coil (Bruker, Model 1P T20063V3; internal diameter 23 mm) for radiofrequency excitation and detection; neither cardiac nor respiratory triggering was applied. Following a short period of introduction in a box, animals were maintained in anesthesia with 1.5% isoflurane (Abbott, Cham, Switzerland) in oxygen, administered via a nose cone. During MRI signal acquisitions, animals were placed in prone position in a cradle made of Plexiglas, the body temperature was kept at 37±1°C using a heating pad, and the respiration was monitored.

A T₂-weighted, two-dimensional multislice RARE (Rapid Acquisition with Relaxation Enhancement) sequence (Hennig et al., 1986) was used for determining the anatomical orientation and for evaluating signal intensities. This was followed by a two-dimensional multislice gradient-recalled FLASH (Fast Low-Angle Shot) acquisition (Haase et al., 1986) for assessment of Magnetization Transfer Ration (MTR). As both sequences had the same anatomical parameters, the choice of the regions-of-interest for evaluations was performed on the RARE images and then transferred to the FLASH images. The parameters of the acquisitions were the following: (a) RARE sequence: effective echo time 80 ms, repetition time 3280 ms, RARE factor 16, 12 averages, field of view 20x18 mm², matrix size 213x192, pixel size 0.094x0.094 mm², slice thickness 0.5 mm, 15 adjacent slices. Hermite pulses of duration/bandwidth 1 ms/5400 Hz and 0.64 ms/5344 Hz were used for radiofrequency excitation and refocusing, respectively. Fat suppression was achieved by a gauss512 pulse of 2.61 ms/1051 Hz duration/bandwidth followed by a 2-ms-long gradient spoiler. The total acquisition time was of 7 min 52.3 s; (b) FLASH sequence: echo time 2.8 ms, repetition time 252.8 ms, 4 averages, field of view 20x18 mm², matrix size 213x192, pixel size 0.094x0.094 mm², slice thickness 0.5 mm, 15 adjacent slices. A hermite pulse of 0.9 ms/6000 Hz duration/bandwidth and flipangle 30° was used for radiofrequency excitation. MTR contrast was introduced by a gauss pulse of 15 ms/182.7 Hz duration/bandwidth applied with a radiofrequency peak amplitude of 7.5 µT and an irradiation offset of 2500 Hz. The acquisition was then repeated with the same parameters but without the introduction of the MTR contrast. MTR was then computed using the formula MTR = (S₀-S_{MTR})/S₀, where S₀ and S_{MTR} represent respectively the signal intensities in the FLASH acquisitions without and with the introduction of the MTR contrast. The total acquisition time for both data sets was of 6 min 31.6 s.

### Histology of brain sections

After fixation brains were processed for paraffin embedding by dehydration through increasing ethanol series. Automated immunohistochemistry of paraffin sections was performed on 3 µm paraffin sections mounted on SuperFrost+ slides and automatically immunostained using the Discovery XT technology (Ventana/Roche Diagnostics). Briefly, sections were deparaffinized, rehydrated, subjected to antigen retrieval by heating with CC1 cell conditioning buffer, incubated for 1-3 hours at room temperature with primary antibody diluted in antibody diluent, incubated with the respective biotinylated secondary antibody diluted in antibody dilution, reacted with DABMab kit and counterstained with Hematoxylin II and Bluing Agent reagent (Ventana/Roche Diagnostics). Slides were washed with soap in hot tap water and rinsed under cold running tap water to remove the soap, then dehydrated and embedded with Pertex.

Manual immunofluorescence staining of paraffin sections was performed on 3 µm paraffin sections. They were de-waxed and subjected to antigen retrieval (exception: GST-π) by microwaving for 15-20 minutes at 98 °C in 0.1 M citrate buffer pH 6.0 (quartett 400300692), the jar with slides were cooled to room temperature for 20-25 min and rinsed in 1xTBS (cell signaling 12498S) twice for 3 min. Endogenous peroxidase was quenched with Perox Block (Dako S2023) for 10 min, sections then washed with 1xTBS twice for 3 min and blocking with 10% normal goat serum (Dako X0907) in 1xTBS for 20 min performed. Sections were then incubated without rinsing with primary antibody for 60 min (MBP) or overnight (GST-pi, MOG) at room temperature. Sections were then washed in 1xTBS twice for 3 min and then incubated with secondary antibody for 30 min at room temperature. Sections were then washed with 1xTBS twice for 3min and incubated with Vectastain ABC Elite Kit (Vector PK6100) for 30 min at room temperature, washed with 1xTBS twice for 3 min, incubated with DAB solution (Dako K3468) for 1-5 min. Sections were finally washed with distilled water twice for 2 min, counterstained with Hematoxylin (Dako S2020) for 3 min, again washed with running tap water for 5 min, dehydrated and embedded in Pertex.

### Antibodies

Primary antibodies and antibody dilutions: Rabbit anti-mouse Myelin Basic Protein (MBP, Dako A0623), 1:1000 in Ventana Antibody Diluent, 1:1500 in Dako Diluent (Dako S2022); Rabbit anti-mouse GST-π (MBL 312), 1:500 in Ventana Antibody Diluent, 1:1000 in Dako Diluent (Dako S2022); Rabbit anti-Iba1 (Wako 019-19741, 50 ug/100ul), 1:500 in Ventana Antibody Diluent; Rabbit anti-GFAP (Dako Z0334), 1:5000 in Ventana Antibody Diluent; Rabbit anti-mouse Myelin oligodendrocyte glycoprotein (MOG, abcam ab32760), 1:100 in Ventana Antibody Diluent, 1:600 in Dako Diluent (Dako S2022)
Secondary detection antibodies: Goat anti-rabbit IgG biotinylated (Jackson ImmunoResearch 111-065-144), 1:1000 in Ventana Antibody Diluent, 1:500 in Dako Diluent; Goat anti-rabbit IgG biotinylated (Vector BA-1000), 1:200 in Dako Diluent; Goat anti-mouse IgG biotinylated (Vector BA-9200), 1:1000 in Ventana Antibody Diluent, 1:200 in Dako Diluent.

### Image analysis

For the quantitative evaluation of microglia/astrocyte numbers based on image analysis from histological stained brain sections, a proprietary image analysis platform (ASTORIA, Automated Stored Image Analysis) was developed based on MS Visual Studio 2010 and many functions from Matrox MIL V9 libraries (Matrox Inc, Quebec, Canada).

For the detection and analysis of soma the following sequence of steps was performed: 1. Slides with brain sections for assessment of (brown) IHC stained microglia (Iba1) or astrocyte (GFAP) were scanned with Aperio's Scanscope at 20x magnification. 2. Run the in-house developed ImageScope (V12.1.0.5029, Aperio Inc., USA) plug-in for creation and export of 3x8-bit RGB tif image tiles (at 20x magnification) for each section. 3. Image processing (for each tif image): A: Perform color deconvolution on each tif image to obtain brown stained image without blue. B: Segmentation of valid sample (brain tissue) from white background through thresholding, morphological closing, filling of holes, opening and elimination of too small objects, resulting in a binary mask of the valid tissue and sample area for the current image. C: Apply an adaptive thresholding technique for the individual segmentation of soma, based on the average gray value of the blue channel of the color-deconvoluted brown image at sufficiently dark regions (indicative for soma). The computed threshold is used for binarization, and after size filtering yielding the soma mask image (within the valid sample region).

### Results

**Figure 1**: 2-week therapeutic treatment with BLZ945 after cuprizone intoxication **reduces MRI contrast in cortex and striatum but not corpus callosum compared to vehicle controls indicative of increased myelination.** Magnetic resonance imaging (MRI) contrast in cortex (Figure 1A), striatum (Figure 1B) and corpus callosum (Figure 1C) for the different treatment groups. MRI contrast is normalized to absolute values of the vehicle control group (normal food). Groups consisted of mice treated for 5 weeks with normal food or cuprizone food (0.2%) and then switched to normal food for the 2-week therapeutic treatment (vehicle or BLZ945 p.o., qd, 169 mg/kg). Magnetization transfer ratio (MTR) measured in the MRI for the different treatment groups in the corpus callosum (Figure 1D). Individual results of two different experiments (grey and black) are shown. Statistics: Turkey's multiple comparison test (**: p<0.01, ***: p<0.001, ****: p<0.0001, n.s.: not significant).

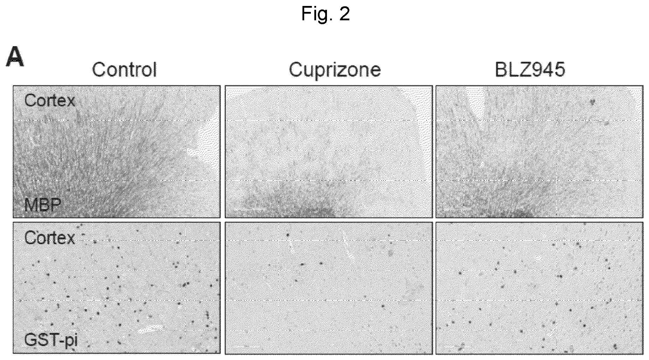

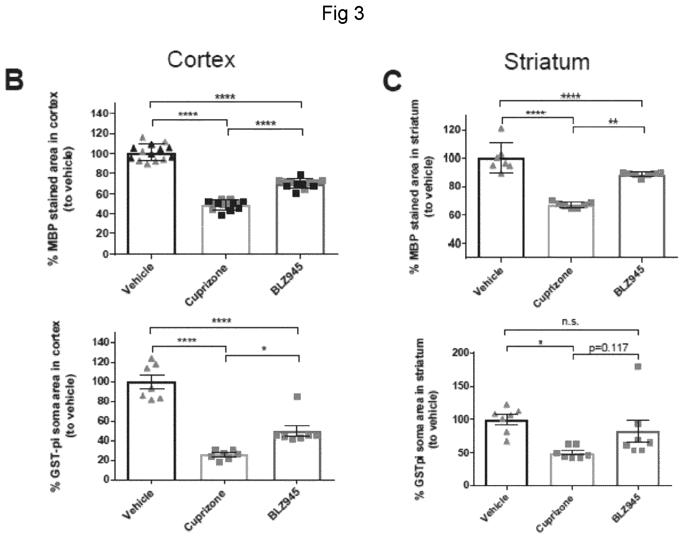

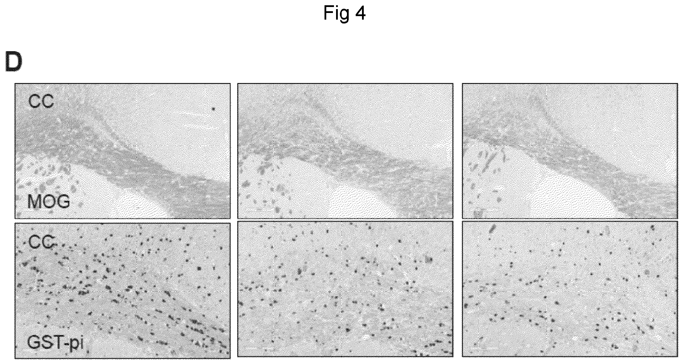

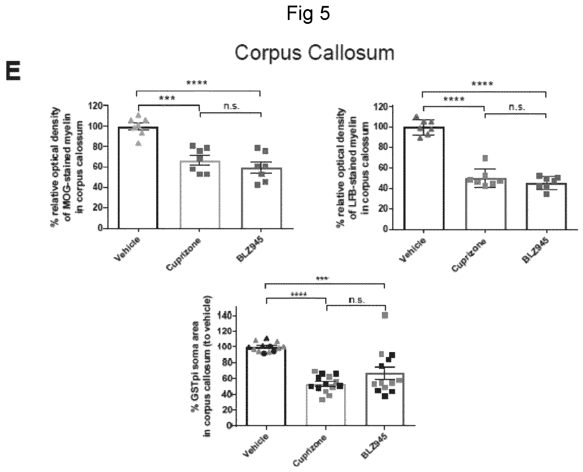

**Figures 2-5**: 2-week therapeutic treatment with BLZ945 after cuprizone intoxication **enhances remyelination and increases oligodendrocyte numbers in cortex and striatum but not corpus callosum compared to vehicle.** Example pictures from immunohistological stainings detecting myelin basic protein (MBP) and mature oligodendrocytes positive for GST-π in the cortex (Figure 2) for the different treatment groups. Groups consisted of mice treated for 5 weeks with normal food or cuprizone food (0.2%) and then switched to normal food for the 2-week therapeutic treatment (vehicle or BLZ945 p.o., qd, 169 mg/kg). Quantitative analysis of the immunohistochemistry for MBP (stained area) and GST-π (number of positive cells) in the cortex (Figure 3A) and striatum (Figure 3B) normalized to control vehicle. Example pictures from immunohistological stainings detecting myelin oligondendrocyte glycoprotein (MOG) and mature oligodendrocytes positive for GST-π in the corpus callosum (Figure 4) for the different treatment groups. Quantitative analysis of the immunohistochemistry for MOG (optical density) and GST-π (number of positive cells) and also optical density analysis of Luxol fast blue (LFB) in the corpus callosum (Figure 5) normalized to control vehicle. In Figure 3A, 3B and Figure 5 individual results of two different experiments (grey and black) are shown. Statistics: Turkey's multiple comparison test (*: p<0.05, **: p<0.01, ***: p<0.001, ****: p<0.0001, n.s.: not significant).

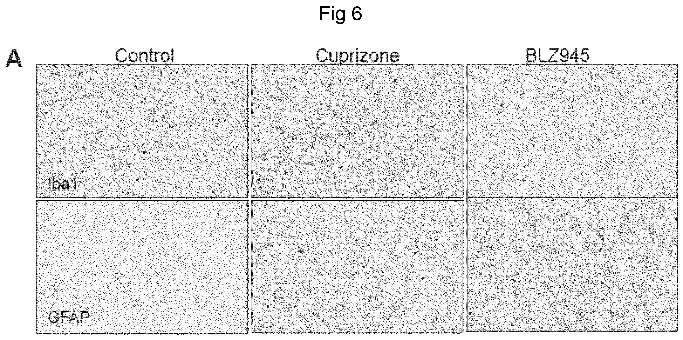

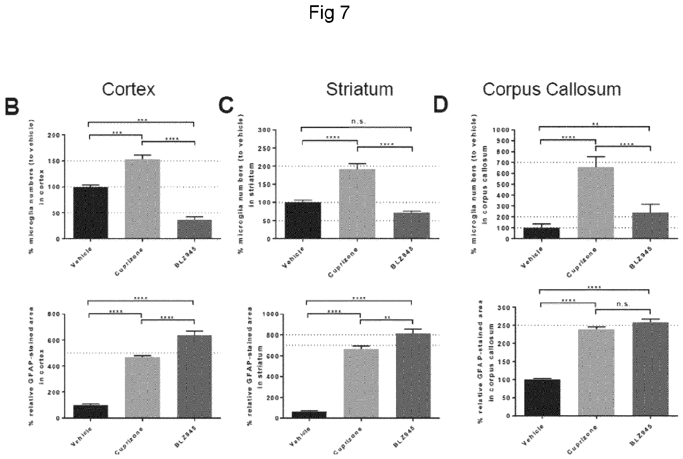

**Figures 6** **and** **7**: 2-week therapeutic treatment with BLZ945 after cuprizone **intoxication reduces microglia numbers but enhances astrocytes.** Example pictures from immunohistological stainings detecting the microglia marker Iba1 and glial fibrillary acidic protein (GFAP) astrocytes in the cortex (Figure 6) for the different treatment groups. Groups consisted of mice treated for 5 weeks with normal food or cuprizone food (0.2%) and then switched to normal food for the 2-week therapeutic treatment (vehicle or BLZ945 p.o., qd, 169 mg/kg). Quantitative analysis of the immunohistochemistry for Iba1-positive microglia numbers and GFAP-positive astrocyte stained area in the cortex (Figure 7A), striatum (Figure 7B) and corpus callosum (Figure 7C) normalized to control vehicle. Statistics: Turkey's multiple comparison test (**: p<0.01, ***: p<0.001, ****: p<0.0001, n.s.: not significant).

The cuprizone model is ideal to analyze remyelination events. We used 0.2 % cuprizone in rodent feed for 5 weeks to induce a strong demyelination together with a massive involvement of microglia and astrocytes. We implemented a longitudinal, non-invasive magnetic resonance imaging (MRI) method to measure directly myelination in the brain and correlated the MRI parameters with quantitative histological readouts in brain. In this model we tested the CSF1R kinase inhibitor BLZ945 at a high dose of 169 mg/kg qd, p.o. that in naive animals almost completely depletes microglia after 5 days in all brain areas.

BLZ945 treatment for 2 weeks with normal feed after induction of demyelination for 5 weeks with 0.2% cuprizone feed showed a significant beneficial effect in the MRI contrast in the cortex and striatum in two independent experiments (Figures 1A, 1B). The MRI contrast almost normalized to levels of control mice, whereas the MRI contrast of cuprizone treated mice still showed a significance difference of MRI contrast to control. This effect was highly significant after 2 weeks of treatment. No beneficial effect of BLZ945 was observed in the corpus callosum in two independent experiments (Figure 1C), both for MRI contrast and magnetization transfer ratio (MTR). The positive effect in the MRI observed after 2 weeks of BLZ945 treatment could be confirmed in immunohistochemistry of paraffin-embedded brain sections and quantitative image analysis. Myelin basic protein (MBP) and oligodendrocytes (GST-π) were significantly increased after BLZ945 treatment compared to vehicle control in the cortex (Figure 2, Figure 3A) and striatum (Figure 3B). However, the extent of MBP-positive remyelination and GST-π-positive oligodendrocyte numbers did not reach control levels after 2 weeks treatment. Furthermore, the absence of any enhancement of remyelination in the corpus callosum as observed by MRI could be verified by histology. There was a marked reduction of myelin oligodendrocyte glycoprotein (MOG), oligodendrocytes (GST-π) as well as Luxol Fast Blue (LFB) after cuprizone treatment compared to control but no difference to 2-weeks BLZ945 treatment (Figure 4, Figure 5).

Microglia and astrocytes are highly activated and increased after 5-week cuprizone-induced demyelination (Figure 6) in all brain areas. In cortex and striatum the numbers of Iba1+ microglia are 1.5-2-fold higher than in control mice (Figures 7A, 7B) whereas in the corpus callosum this increase is 6-fold higher (Figure 7C). BLZ945 is significantly reducing this increase compared to vehicle treated animals in all brain areas analyzed (Figures 7A, 7B, 7C). Moreover, BLZ945 treated mice showed a lower number of Iba1+ microglia in cortex and striatum compared to control (Figures 7A, 7B) but not in the corpus callosum (Figure 7C). In cortex and striatum the GFAP-positive astrocytes are increased 5-6-fold compared to control (Figures 7A, 7B) whereas in the corpus callosum this increase is only 2-2.5-fold higher (Figure. 7C). Interestingly, the extent of increase of microglia and astrocyte in cortex, striatum and corpus callosum seem to occur reciprocally. In cortex and striatum the increase of microglia (1.5-2-fold) are not as extensive as the increase of astrocytes (4-6-fold) whereas in the corpus callosum it's the opposite, the increase of Iba-positive microglia is dramatic (6-fold) than that of astrocytes (2.5-fold). However, BLZ945 treatment for 2 weeks even further increases astrocyte numbers in all brain areas compared to vehicle (Figure 6 and Figures 7A-C). This astrocyte increase is substantially higher in cortex and striatum than it is in the corpus callosum.

### Example 2 MOG peptide-induced EAE

Studies described below were approved by the Swiss Cantonal Veterinary Authority of Basel City, Switzerland, under the license number 2119.

Mice (C57BL/6J OlaHsd) were commercially purchased from Harlan Laboratories BV (9 weeks old, females). All the animals were allowed to adapt for 7 days prior the start of the experiment and housed in IVC racks (max. 4 mice/XJ Type cage). The animals were given access to food and water ad libitum.

Mice were immunized at with subcutaneous injection on the lower back of myelin oligodendrocyte glycoprotein peptide (MOG1-125; in house produced; 200 µg/100 µl) emulsified in 4 mg/ml complete Freund adjuvant (CFA, Sigma). Pertussis toxin (Fluka; 200 ng per mouse) was administered intraperitoneally on day 0 and day 2. Evaluation of EAE: The mice were observed and weighed daily. They were assessed for clinical signs on a scale from 0 to 5, with graduations of 0.5 for intermediate scores. 0: no clinical signs, 1: Limp tail / complete loss of tail tonus; 2: Clear hind limb weakness; 3: Complete bilateral hind limb paralysis; 4: Fore limbs and hind limbs paralysis; 5: Death. Supplementary food and gel food were provided inside the cage once first clinical signs appeared.

Animals were treated for 14 days with 100 and 150 mg/kg of BLZ945, qd, p.o. 10ml/kg from day 14 to day 28 post-immunization. BLZ945 was prepared in 0.5% methylcellulose in water and 0.1% Tween-80.

### Results

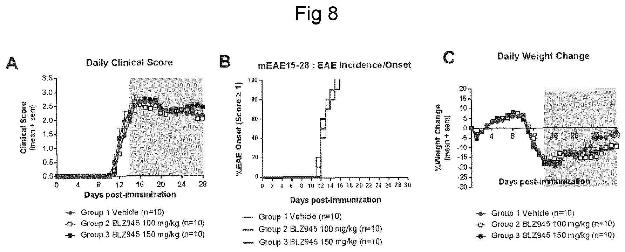

**Figure 8****: Therapeutic BLZ945 treatment in experimental autoimmune encephalomyelitis (EAE) mice did not alter disease progression.** Evaluation of the clinical score after myelin oligodendrocyte glycoprotein peptide-immunization revealed similar disease progression (Figure 8A), disease onset/incidences (Figure 8B) and weight changes (Figure 8C) for all treatment groups. BLZ945 treatment started 14 days post-immunization and a near maximum clinical score until for 14 days (grey shaded area). Experiment ended at 28 days post-immunization.

To investigate the relevance of microglia in an autoimmune disease model we therapeutically treated experimental autoimmune encephalomyelitis (EAE) mice, a model of human MS. Mice were treated at two different doses (100 and 150 mg/kg, p.o., qd) starting at near maximal disease 14 days after immunization. Treatment lasted until the end at day 28 post-immunization. No difference in clinical score for both treatment groups compared to vehicle could be observed (Figure 8A). The onset of EAE clinical pathology around 11-12 days post-immunization (Figure 8B) as well as the weight change (Figure 8C) was similar between the treatment and vehicle groups. Taken together, CSF1R kinase inhibition does not alter disease progression in the EAE model, a peripheral driven neuroinflammation model.

The above data from Examples 1 and 2 suggest that inhibition of the CSF-1 receptor pathway in a therapeutic mode enhances central remyelination repair mechanisms by modulating neuroinflammation and thus is beneficial for treating demyelinating and neuroinflammatory diseases such multiple sclerosis, amyotrophic lateral sclerosis and Alzheimer's disease. The colony-stimulating factor 1 receptor (CSF1R) signaling pathway is essential for microglia survival, the resident phagocytes of the brain. Microglia play an important role in modeling the CNS synaptic circuitry and removal of pathogens and cell debris, of which the latter is essential to initiate repair by remyelination. Thus, by inhibition of the CSF-1 receptor pathway the inventor did not expect a beneficial effect in the cuprizone model as these phagocytes are missing to remove myelin debris. Other groups have shown that inhibition of the CSF1R pathway is also neuroprotective in disease conditions like amyotrophic lateral sclerosis and Alzheimer's disease by reducing chronic neuroinflammation. However, chronically activated microglia seem to be neurotoxic, whereas in the cuprizone condition microglia are activated for a short-time to fulfill a certain directed function, like phagocytosis. Nevertheless, it seems that microglia in the cuprizone model seem to surprisingly have additionally a neurotoxic component and that by removing microglia with BLZ945 this microglia toxicity in the resolution phase is eliminated. Additionally, astrocytes are activated after BLZ945 treatment, which seem to take over the phagocytic activity and exert certain trophism for oligodendrocytes without the neurotoxic component of microglia. Furthermore, microglia seem to be involved in the differentiation processes of oligodendrocyte precursor cells OPC) to oligodendrocytes (OD) (Hagemeyer et al., 2017). By removing microglia there seems to be a prompt differentiation processes initiated that facilitates NG2-positive OPCs to differentiate to ODs thereby enhancing remyelination.

### Example 3 : Cuprizone-induced demyelination in mice at 20mg/kg and 60 mg/kg.

Studies described below were approved by the Swiss Cantonal Veterinary Authority of Basel City, Switzerland, under the license number 2711. Mice (C57BL/6J) were commercially purchased from Charles River Laboratories, Germany or obtained from Novartis Pharma AG breeding colonies (8-9 weeks old, females). All the animals were allowed to adapt for 7 days prior the start of the experiment and housed in IVC racks (max. 4 mice/XJ Type cage). The animals were given access to food and water ad libitum. Animals were treated with cuprizone for 5 weeks. Cuprizone (Bis(cyclohexanone)oxaldihydrazone, Sigma-Aldrich) was mixed into rodent food pellets (0.2% w/w) by Provimi Kliba AG, Switzerland. Therapeutic treatment of animals after cuprizone was performed for 2 weeks on normal food with 20 and 60 mg/kg of BLZ945, qd, p.o. 10ml/kg. BLZ945 was prepared in vehicle consisting of 0.5% methylcellulose in water and 0.1% Tween-80. Before killing 3h after last BLZ945 dosing animals were perfused transcardially by phosphate-buffered saline (PBS), brains isolated and fixed in 4% paraformaldehyde (PFA) for 48h at 4°C.

### Magnetic Resonance Imaging (MRI)

Measurements were performed with a Biospec 70/30 spectrometer (Bruker Medical Systems, Ettlingen, Germany) operating at 7 T. The operational software of the scanner was Paravision 5.1 (Bruker). Images were acquired from anesthetized, spontaneously breathing animals using a mouse brain circularly polarized coil (Bruker, Model 1P T20063V3; internal diameter 23 mm) for radiofrequency excitation and detection; neither cardiac nor respiratory triggering was applied. Following a short period of introduction in a box, animals were maintained in anesthesia with 1.5% isoflurane (Abbott, Cham, Switzerland) in oxygen, administered via a nose cone. During MRI signal acquisitions, animals were placed in prone position in a cradle made of Plexiglas, the body temperature was kept at 37±1°C using a heating pad, and the respiration was monitored.

A T₂-weighted, two-dimensional multislice RARE (Rapid Acquisition with Relaxation Enhancement) sequence (Hennig et al., 1986) was used for determining the anatomical orientation and for evaluating signal intensities. This was followed by a two-dimensional multislice gradient-recalled FLASH (Fast Low-Angle Shot) acquisition (Haase et al., 1986) for assessment of MTR. As both sequences had the same anatomical parameters, the choice of the regions-of-interest for evaluations was performed on the RARE images and then transferred to the FLASH images.

The parameters of the acquisitions were the following: (a) RARE sequence: effective echo time 80 ms, repetition time 3280 ms, RARE factor 16, 12 averages, field of view 20x18 mm², matrix size 213x192, pixel size 0.094x0.094 mm², slice thickness 0.5 mm, 15 adjacent slices. Hermite pulses of duration/bandwidth 1 ms/5400 Hz and 0.64 ms/5344 Hz were used for radiofrequency excitation and refocusing, respectively. Fat suppression was achieved by a gauss512 pulse of 2.61 ms/1051 Hz duration/bandwidth followed by a 2-ms-long gradient spoiler. The total acquisition time was of 7 min 52.3 s; (b) FLASH sequence: echo time 2.8 ms, repetition time 252.8 ms, 4 averages, field of view 20x18 mm², matrix size 213x192, pixel size 0.094x0.094 mm², slice thickness 0.5 mm, 15 adjacent slices. A hermite pulse of 0.9 ms/6000 Hz duration/bandwidth and flipangle 30° was used for radiofrequency excitation. MTR contrast was introduced by a gauss pulse of 15 ms/182.7 Hz duration/bandwidth applied with a radiofrequency peak amplitude of 7.5 µT and an irradiation offset of 2500 Hz. The acquisition was then repeated with the same parameters but without the introduction of the MTR contrast. MTR was then computed using the formula MTR = (S₀-S_{MTR})/S₀, where S₀ and S_{MTR} represent respectively the signal intensities in the FLASH acquisitions without and with the introduction of the MTR contrast. The total acquisition time for both data sets was of 6 min 31.6 seconds.

### Quantitative determination of BLZ945 in blood and brain

Sample preparation and analysis was based on a modified protein precipitation procedure followed by liquid chromatographic separation coupled with mass spectrometry for detection. Cerebella were homogenized in a gentleMACS^{™} Dissociator (Milteniy Biotec, # 130-093-235) by adding 20 % CH₃OH to a final concentration of 0.20 g/mL. Blood-EDTA samples were directly used for further preparation. Calibration, quality control, and recovery control samples were prepared by spiking blank blood and blank brain homogenate with known quantities of BLZ945 (between 0.02 and 62.5 µg/mL). For analyte determination, Labetalol hydrochloride (Sigma-Aldrich, #L1011) was used as generic internal standard (IS). Aliquots of 10 µL calibration standard, quality control, recovery control, and unknown samples were transferred to 0.75 ml 96- well-Loborack (Vitaris AG, # 51004BC -MIC) and 3 µL IS mixture (2.5 µg/mL in 50 % CH₃CN) was added to each tube. For protein precipitation and extraction from the blood and brain matrix, 200 µl CH₃CN was added. After vortexing for 10 min, the samples were centrifuged at 3220 g for 15 min at 4°C. 50 µl of the upper layer was transferred to a 1.2 mL 96 deep well plate (Thermo Scientific, # AB-0787).

LC-MS-MS analysis: For quantitative analysis, a 1.5 µl aliquot of each sample, including calibration, quality control, and recovery control samples were injected with a cooled Waters^{™} Acquity Urbinary sample manager and HPLC system. The test article and its internal standard were separated with an ACE C18-PFP (50 x 2.1 mm ID, 3.0 µm pore size; # ACE-1110-0502) as column at 40°C. For separation a linear gradient from 5 to 65 % B in 1.7 min at a flow rate of 0.400 mL/min was applied. The total cycle time was 3.5min. The mobile phase used was A: water with 0.1 % formic acid, and B: CH₃CN with 0.1 % formic acid?. For detection the column effluent was directly guided in a AB Sciex API5500 Triple quad mass spectrometer equipped with a TurboIonSpray^{™} interface. The detection was done in multiple reaction monitoring (MRM) positive ion mode. Quantification was based on the compound/IS ratio of the extracted ion chromatograms of the selected mass transitions 399 m/z → 242 m/z for BLZ945 and 329 m/z → 162 m/z for Labetalol (IS). The unknown sample concentration was calculated using external calibration curves. The Lower Limit Of Quantification (LLOQ) of the method was set to 20 ng/mL for blood, and 100 ng/mL for brain samples, and the recovery from the matrix was 95±2 %. All calculations were performed with AB Sciex Analyst software 1.6.2.

### Histology of brains

After fixation brains were processed for paraffin embedding by dehydration through increasing ethanol series. Automated immunohistochemistry of paraffin sections was performed on 3 µm paraffin sections mounted on SuperFrost+ slides (Thermo Fisher Scientific) and automatically immunostained using the Discovery XT technology (Ventana, Roche Diagnostics). Sections were deparaffinized, rehydrated, subjected to antigen retrieval by heating with CC1 cell conditioning buffer for 28-68 min according to the antibody, incubated for 1-3 hours according to the antibody at room temperature with primary antibody diluted in antibody diluent (Ventana), incubated with the respective biotinylated secondary antibody diluted in antibody diluent, reacted with DABMab kit and counterstained with Hematoxylin II and Bluing reagent (Ventana). Slides were washed with soap in hot tap water and rinsed under cold running tap water to remove the soap, then dehydrated and embedded with Pertex.

### Antibodies

Primary antibodies are: Rabbit anti-Iba1 (Wako 019-19741, 50 µg/100 µl) 1:500; rabbit anti-GFAP (Dako Z0334) 1:5000
Secondary detection antibodies are: Goat anti-rabbit IgG biotinylated (Jackson ImmunoResearch 111-065-144) 1:1000; Goat anti-rabbit IgG biotinylated (Vector BA-1000) 1:200 or 1:1000

### Image analysis

For the quantitative evaluation of microglia/astrocyte numbers based on image analysis from histological stained brain sections, a proprietary image analysis platform (ASTORIA, Automated Stored Image Analysis) was developed based on MS Visual Studio 2010 and many functions from Matrox MIL V9 libraries (Matrox Inc, Quebec, Canada).

For the detection and analysis of soma the following sequence of steps was performed: 1. Slides with brain sections for assessment of (brown) IHC stained microglia (Iba1) or astrocyte (GFAP) were scanned with Aperio's Scanscope at 20x magnification. 2. Ran the in-house developed ImageScope (V12.1.0.5029, Aperio Inc., USA) plug-in for creation and export of 3x8-bit RGB tif image tiles (at 20x magnification) for each section. 3. Image processing (for each tif image): A: Performed color deconvolution on each tif image to obtain brown stained image without blue. B: Segmentation of valid sample (brain tissue) from white background through thresholding, morphological closing, filling of holes, opening and elimination of too small objects, resulting in a binary mask of the valid tissue and sample area for the current image. C: Applied an adaptive thresholding technique for the individual segmentation of soma, based on the average gray value of the blue channel of the color-deconvoluted brown image at sufficiently dark regions (indicative for soma). The computed threshold was used for binarization, and after size filtering yielding the soma mask image (within the valid sample region).

### Results

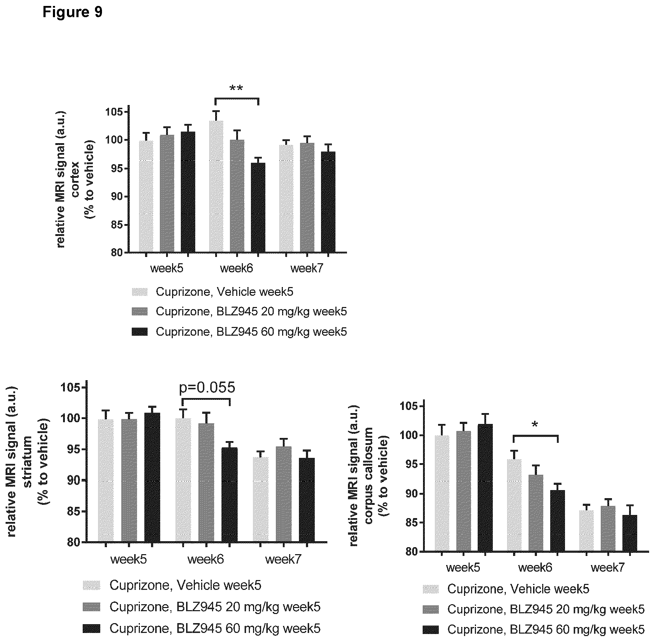

**Figure 9**: 2-week therapeutic treatment with BLZ945 after cuprizone intoxication **reduces MRI contrast in cortex and striatum but not corpus callosum compared to vehicle controls indicative of increased myelination.** Magnetic resonance imaging (MRI) contrast in cortex (Figure 9A), striatum (Figure 9B) and corpus callosum (Figure 9C) for the different treatment groups. MRI contrast is normalized to absolute values of the vehicle control group (normal food). Groups consisted of mice treated for 5 weeks with normal food or cuprizone food (0.2%) and then switched to normal food for the 2-week therapeutic treatment (vehicle or BLZ945 p.o., qd, 20 1nd 60 mg/kg). Mean±SEM. Statistics: Holm Sidak's multiple comparison test (**: p<0.01, **: p<0.005).

**Figure 10****: BLZ945 treatment leads to microglia depletion and microglia activation in a dose-dependent manner.** Dose-dependent microglia depletion (Iba1-positive microglia numbers) in the cortex of wildtype mice after 5 days of treatment with BLZ945 (p.o., qd) (Figure 10A). 60 mg/kg BLZ945 showed only a minor reduction of less than 50%, whereas 20 mg/kg BLZ945 showed no microglia depletion. Dose-dependent microglia activation (Iba1-positive microglia size normalized to distal microglia processes) in the cortex of wildtype mice after 5 days of treatment with BLZ945 (p.o., qd) (Figure 10B). 60 mg/kg BLZ945 induced strong microglia activation, whereas 20 mg/kg BLZ945 only mildly. Individual data points are indicated. Mean±SEM.

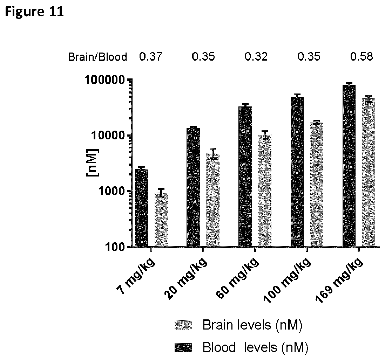

**Figure 11****: Pharmacokinetic analysis of BLZ945 in blood and brain.** The blood and brain BLZ945 levels were dose-dependent and showed a brain/blood ratio of 0.3-0.6 as indicated above the respective graphs. Group sizes: n= 2-3. Data is shown as mean±SEM.

0.2 % cuprizone was used in rodent feed for 5 weeks to induce a strong demyelination together with a massive involvement of microglia and astrocytes. A longitudinal, non-invasive magnetic resonance imaging (MRI) method was implemented to measure directly myelination in the brain. It was shown previously that the MRI parameters correlated with quantitative histological readouts in brain. In this model the CSF1R kinase inhibitor BLZ945 was tested at doses of 20 and 60 mg/kg qd, p.o.. In naive animals the lower dose of BLZ945 after 5-day treatment showed no microglia reduction, but a slight increase in the activation status as measured morphologically. The 60 mg/kg dose of BLZ945 after 5-day treatment in naive animals reduced microglia about 30-40% but also substantially enhanced the morphological activation status of the microglia. These changes in microglia were very different to those observed with 169 mg/kg BLZ945 after 5-day treatment, as with the high dose near complete microglia depletion could be observed. Thus, 20 and 60 mg/kg BLZ945 were tested in the therapeutic mode in the cuprizone model. Short-term treatments with lower doses of BLZ945 for only 1 week showed beneficial effects on remyelination in the 5-week rodent cuprizone model as detected by MRI. The 60 mg/kg BLZ945 dose showed significantly enhanced remyelination in corpus callosum/external capsule and in the cortex, while only a trend in the striatum. These data show that the altered microglia morphology observed in naive animals with 60 mg/kg BLZ945 could lead to an enhanced phagocytosis (engulfing of solid particles/debris/dead cells) and clearance of myelin debris as well as increasing oligodendrocytes by releasing factors for enhanced oligodendrocyte precursor cell differentiation. Furthermore, the mildly reduced microglia numbers with 60 mg/kg BLZ945 dose in combination with the altered microglia phenotype could protect neurons/axons of being damaged in an inflammatory milieu like in MS.

CSF1R kinase inhibitors also effect Kupffer cells in the liver and thereby alter the liver enzymes. This on target effect has to be closely monitored in the clinic and also limits dose and treatment regimen. Thus, the observed beneficial effect of lower BLZ945 doses with only short-term treatment paradigms in preclinical models are highly advantageous. Furthermore, similar compound exposure in the clinic and in the preclinical models with the selected doses are expected.
Calabrese, M., Magliozzi, R., Ciccarelli, O., Geurts, J.J.G., Reynolds, R., and Martin, R. (2015). Exploring the origins of grey matter damage in multiple sclerosis. Nat. Rev. Neurosci. 16, 147-158.
Haase, A., Matthaei, D., Hanicke, W., and Frahm, J. (1986). Dynamic digital subtraction imaging using fast low-angle shot MR movie sequences. Radiology 160, 537-541.
Hagemeyer, N., Hanft, K.-M., Akriditou, M.-A., Unger, N., Park, E.S., Stanley, E.R., Staszewski, O., Dimou, L., and Prinz, M. (2017). Microglia contribute to normal myelinogenesis and to oligodendrocyte progenitor maintenance during adulthood. Acta Neuropathol.
Hennig, J., Nauerth, A., and Friedburg, H. (1986). RARE imaging: A fast imaging method for clinical MR. Magn. Reson. Med. 3, 823-833.
Nasrabady, S.E., Rizvi, B., Goldman, J.E., and Brickman, A.M. (2018). White matter changes in Alzheimer ' s disease : a focus on myelin and oligodendrocytes. 1-10.
Nylander, A., and Hafler, D.A. (2012). Multiple sclerosis. J. Clin. Invest. 122, 1180-1188.
Oliveira Santos, M., Caldeira, I., Gromicho, M., Pronto-Laborinho, A., and de Carvalho, M. (2017). Brain white matter demyelinating lesions and amyotrophic lateral sclerosis in a patient with C9orf72 hexanucleotide repeat expansion. Mult. Scler. Relat. Disord. 17, 1-4.
Ropele, S., Schmidt, R., Enzinger, C., Windisch, M., Martinez, N.P., and Fazekas, F. (2012). Longitudinal magnetization transfer imaging in mild to severe Alzheimer disease. AJNR Am J Neuroradiol 33, 570-575.
Zhou, T., Ahmad, T.K., Gozda, K., Truong, J., Kong, J., and Namaka, M. (2017). Implications of white matter damage in amyotrophic lateral sclerosis (review). Mol. Med. Rep. 16, 4379-4392.

1. A method for the treatment of multiple sclerosis comprising the administration of an effective amount of a compound of formula (I) 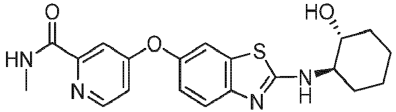 or a pharmaceutically acceptable salt thereof, to a subject in need thereof.
2. The method according to embodiment 1 wherein the subject is a primate.
3. The method according to embodiment 2 wherein the subject is a human.
4. The method according to any one of embodiments 1 to 3 wherein the compound of formula (I) is administered as a free base.
5. The method according to any one of embodiments 1 to 3 wherein the pharmaceutically acceptable salt is HCl.
6. The method according to any one of embodiments 1 to 3 wherein the multiple sclerosis is clinically isolated syndrome.
7. The method according to any one of embodiments 1 to 3 wherein the multiple sclerosis is relapsing-remitting multiple sclerosis.
8. The method according to any one of embodiments 1 to 3 wherein the multiple sclerosis is primary progressive multiple sclerosis or secondary progressive multiple sclerosis.
9. A method for the treatment of multiple sclerosis comprising administration of 4-(2-((1R,2R)-2-hydroxycyclohexylamino)benzothiazol-6-yloxy)-N-methylpicolinamide or a pharmaceutically acceptable salt thereof to a human in need of treatment thereof wherein 4-(2-((1R,2R)-2-hydroxycyclohexylamino)benzothiazol-6-yloxy)-N-methylpicolinamide or a pharmaceutically acceptable salt thereof is administered at a dose of about 250 mg to about 350 mg per day.
10. The method according to embodiment 9 wherein the dose is about 275 mg to about 325 mg per day.
11. The method according embodiment 10 wherein the dose is about 300 mg per day.
12. The method according to any one of embodiments 9 to 11 wherein 4-(2-((1R,2R)-2-hydroxycyclohexylamino)benzothiazol-6-yloxy)-N-methylpicolinamide or a pharmaceutically acceptable salt thereof is administered for 4 to 6 consecutive days and then administration of -(2-((1R,2R)-2-hydroxycyclohexylamino)benzothiazol-6-yloxy)-N-methylpicolinamide or a pharmaceutically acceptable salt thereof ceases.
13. The method according to embodiment 12 wherein 4-(2-((1R,2R)-2-hydroxycyclohexylamino)benzothiazol-6-yloxy)-N-methylpicolinamide or a pharmaceutically acceptable salt thereof is administered for 4 consecutive days and then administration of 4-(2-((1R,2R)-2-hydroxycyclohexylamino)benzothiazol-6-yloxy)-N-methylpicolinamide or a pharmaceutically acceptable salt thereof ceases.
14. The method according to embodiment 12 or 13 wherein administration of 4-(2-((1R,2R)-2-hydroxycyclohexylamino)benzothiazol-6-yloxy)-N-methylpicolinamide or a pharmaceutically acceptable salt thereof starts when the human is first diagnosed with multiple sclerosis.
15. A method for the treatment of multiple sclerosis comprising the administration of an effective amount of 4-(2-((1R,2R)-2-hydroxycyclohexylamino)benzothiazol-6-yloxy)-N-methylpicolinamide or a pharmaceutically acceptable salt thereof in combination with another therapeutic agent, to a subject in need thereof.
16. The method according to embodiment 15 wherein 4-(2-((1R,2R)-2-hydroxycyclohexylamino)benzothiazol-6-yloxy)-N-methylpicolinamide or a pharmaceutically acceptable salt thereof is administered separately or by the same or different route of administration as the other agent.
17. The method according to Embodiment 16 wherein the other therapeutic agent is a corticosteroid, a beta interferon, ocrelizumab. glatiramer acetate, dimethyl fumarate, fingolimod, teriflunomide, natalizumab, alemtuzumab or mitoxantrone.
18. The use of a compound of formula (I) 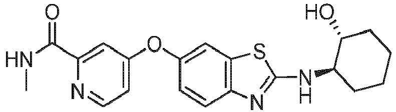 or a pharmaceutically acceptable salt thereof in in the treatment of multiple sclerosis.
19. A compound of formula (I) 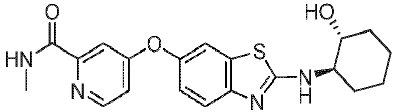 or a pharmaceutically acceptable salt thereof for use in the treatment of multiple sclerosis.
20. The use according to embodiment 11 wherein therapy is a disease selected from multiple sclerosis.

## Claims

1. A compound of formula (I) 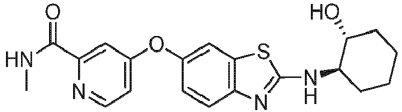 or a pharmaceutically acceptable salt thereof, for use in the treatment of amyotrophic lateral sclerosis comprising administration of 4-(2-((1R,2R)-2-hydroxycyclohexylamino)benzothiazol-6-yloxy)-N-methylpicolinamide or a pharmaceutically acceptable salt thereof to a human in need of treatment thereof wherein 4-(2-((1R,2R)-2-hydroxycyclohexylamino)benzothiazol-6-yloxy)-N-methylpicolinamide or a pharmaceutically acceptable salt thereof is administered at a dose of about 250 mg to about 350 mg per day.

2. The compound for use according to claim 1 wherein the compound of formula (I) is administered as a free base.

3. The compound for use according to claim 1 wherein the pharmaceutically acceptable salt is HCl.

4. The compound for use according to any one of claims 1-3 wherein the dose is about 275 mg to about 325 mg per day, optionally wherein the dose is about 300 mg per day.

5. The compound for use according to any one of claims 1-4 wherein 4-(2-((1R,2R)-2-hydroxycyclohexylamino)benzothiazol-6-yloxy)-N-methylpicolinamide or a pharmaceutically acceptable salt thereof is administered for 4 to 6 consecutive days and then administration of 4-(2-((1R,2R)-2-hydroxycyclohexylamino)benzothiazol-6-yloxy)-N-methylpicolinamide or a pharmaceutically acceptable salt thereof ceases.

6. The compound for use according to claim 5 wherein 4-(2-((1R,2R)-2-hydroxycyclohexylamino)benzothiazol-6-yloxy)-N-methylpicolinamide or a pharmaceutically acceptable salt thereof is administered for 4 consecutive days and then administration of 4-(2-((1R,2R)-2-hydroxycyclohexylamino)benzothiazol-6-yloxy)-N-methylpicolinamide or a pharmaceutically acceptable salt thereof ceases.
